# EUROPEAN PATENT APPLICATION

(11) **EP 1 953 159 A1**
(43) Date of publication of application: **06.08.2008**
(21) Application number: 07101742.0
(22) Date of filing: 05.02.2007
(51) Int. Cl.: C07D 471/04, A61K 31/437, A61P 11/00, A61P 15/00, A61P 9/00, A61P 25/00, A61P 29/00

(54) **6-Benzyl-2,3,4,7-tetrahydro-indolo[2,3-c]quinoline compounds useful as PDE5 inhibitors**

(71) Applicant: Nycomed GmbH, 78467 Konstanz (DE)
(72) Inventor: Weinbrenner, Steffen, 78465 Konstanz (DE); Dunkern, Torsten, 78269 Volkertshausen (DE); Marx, Degenhard, 78345 Moos (DE); Schmidt, Beate, 78476 Allensbach (DE); Stengel, Thomas, 78464 Konstanz (DE); Flockerzi, Dieter, 78476 Allensbach (DE); Kautz, Ulrich, 78476 Allensbach (DE); Hauser, Daniela, 78224 Singen (DE); Diefenbach, Jörg, 78333 Stockach-Winterspüren (DE); Christiaans, Johannes A. M., 8245 DG Lelystad (NL); Menge, Wiro M. P. B., 6821 Hm Arnhem (NL)
(74) Representative: Mechnich, Oliver M.J.

(57) **Abstract**

The compounds of formula (I) 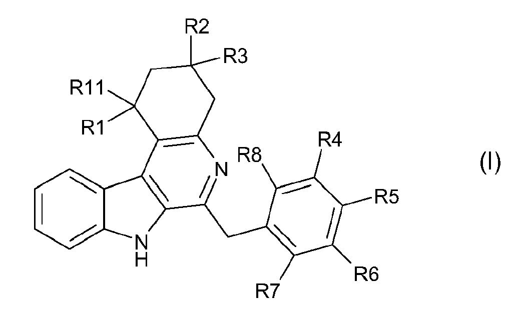 wherein R1 to R8 and R11 have the meanings as given in the description, the salts thereof, the N-oxides of the compounds and the salts thereof, and the stereoisomers of the compounds, the salts, the N-oxides of the compounds and the N-oxides of the salts thereof are effective inhibitors of the type 5 phosphodiesterase.

## Description

### Field of application of the invention

The invention relates to 6-benzyl-2,3,4,7-tetrahydro-indolo[2,3-c]quinoline compounds, which are used in the pharmaceutical industry for the manufacture of pharmaceutical compositions.

### Known technical background

6-Benzyl-3,3-dimethyl-2,3,4,7-tetrahydro-indolo[2,3-c]quinolin-1-one is described in Khimiya Geterotsiklicheskikh Soedinenii (1985) 3, 363-6 without mentioning any pharmaceutical activity thereof.

### Description of the invention

It has now been found that the 6-benzyl-2,3,4,7-tetrahydro-indolo[2,3-c]quinoline compounds, which are described in detail below, have surprising and advantageous properties.

The invention relates to compounds of formula (I) 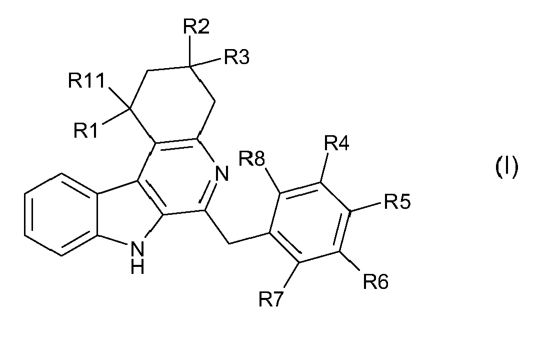 wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
   R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
   under the proviso that the compound 6-benzyl-3,3-dimethyl-2,3,4,7-tetrahydro-indolo[2,3-c]quinolin-1-one is disclaimed;
   a salt thereof, an N-oxide of the compound or the salt thereof, or a stereoisomer of the compound, the salt, the N-oxide of the compound or the N-oxide of the salt thereof.

1-3C-Alkyl is a straight-chain or branched alkyl group having 1 to 3 carbon atoms. Examples are methyl, ethyl, n-propyl and iso-propyl.

Halogen includes fluorine, chlorine, bromine and iodine, with fluorine, chlorine and bromine being preferred.

1-3C-Alkoxy represents a group which, in addition to the oxygen atom, contains a straight-chain or branched alkyl radical having 1 to 3 carbon atoms. Examples are the methoxy, ethoxy, n-propoxy and iso-propoxy group.

The group -NH-C(O)-1-2C-alkyl is selected from -NH-C(O)-CH₃ and -NH-C(O)-C₂H₅.

The methoxy group substituted by 2 or 3 fluorine atoms represents a group selected from a difluoromethoxy group and a trifluoromethoxy group.

The nitro group represents the moiety -NO₂ the amino group represents the moiety -NH₂ and the oxo group represents the moiety =O.

In a preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
under the proviso that at least one of substituents R1 to R8 and R11 is not hydrogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
under the proviso that at least two of substituents R1 to R8 and R11 are not hydrogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
under the proviso that at least one of substituents R4 to R8 is not hydrogen;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
under the proviso that at least two of substituents R4 to R8 are not hydrogen;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is hydroxy;
R11 is hydrogen;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is hydrogen;
R11 is hydrogen;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl and -NH-C(O)-NH₂;
R6 is selected from the group consisting of hydrogen and halogen;
R7 is selected from the group consisting of hydrogen and halogen;
R8 is selected from the group consisting of hydrogen and halogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen and 1-3C-alkoxy;
R5 is selected from the group consisting of hydrogen, halogen, 1-3C-alkyl, hydroxy and 1-3C-alkoxy;
R6 is selected from the group consisting of hydrogen and halogen;
R7 is selected from the group consisting of hydrogen and halogen;
R8 is selected from the group consisting of hydrogen and halogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen and halogen;
R5 is selected from the group consisting of hydrogen, hydroxy and 1-3C-alkoxy;
R6 is selected from the group consisting of hydrogen and halogen;
R7 is selected from the group consisting of hydrogen and halogen;
R8 is hydrogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and methyl;
R3 is selected from the group consisting of hydrogen and methyl;
R4 is selected from the group consisting of hydrogen and fluorine;
R5 is methoxy;
R6 is selected from the group consisting of hydrogen and fluorine;
R7 is selected from the group consisting of hydrogen and fluorine;
R8 is hydrogen;
a salt thereof, an N-oxide of the compound or the salt thereof or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is halogen;
R5 is hydrogen;
R6 is halogen;
R7 is hydrogen;
R8 is hydrogen;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is halogen;
R5 is 1-3C-alkoxy;
R6 is hydrogen;
R7 is hydrogen;
R8 is hydrogen;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is hydrogen;
R5 is 1-3C-alkoxy;
R6 is halogen;
R7 is halogen;
R8 is hydrogen;
a salt thereof, an N-oxide of the compound or the salt thereof, or a stereoisomer of the compound, the salt, the N-oxide of the compound or the N-oxide of the salt thereof.

In a further preferred embodiment, the invention relates to compounds of formula (I), wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
a salt thereof, an N-oxide of the compound or the salt thereof, or a stereoisomer of the compound, the salt, the N-oxide of the compound or the N-oxide of the salt thereof.

It is to be understood that the invention covers all combinations of substituent groups referred to hereinabove. In particular, the invention covers all combinations of preferred groups described herein.

Salts of the compounds according to the invention, the N-oxides thereof, the stereoisomers of the salts and the N-oxides thereof include all inorganic and organic acid addition salts and salts with bases, especially all pharmaceutically acceptable inorganic and organic acid addition salts and salts with bases, particularly all pharmaceutically acceptable inorganic and organic acid addition salts and salts with bases customarily used in pharmacy.

Examples of acid addition salts include, but are not limited to, hydrochlorides, hydrobromides, phosphates, nitrates, sulfates, acetates, trifluoroacetates, citrates, D-gluconates, benzoates, 2-(4-hydroxybenzoyl)benzoates, butyrates, sulfosalicylates, maleates, laurates, malates, lactates, fumarates, succinates, oxalates, tartarates, stearates, benzenesulfonates (besilates), toluenesulfonates (tosilates), methanesulfonates (mesilates), laurylsulfonates, 3-hydroxy-2-naphthoates, lactobionates, galactarates, embonates and ascorbates.

Examples of salts with bases include, but are not limited to, lithium, sodium, potassium, calcium, aluminum, magnesium, titanium, ammonium, meglumine and guanidinium salts.

The salts include water-insoluble and, particularly, water-soluble salts.

The compounds according to the invention, the salts thereof, the N-oxides of the compounds and the salts thereof and the stereoisomers of the compounds, salts, N-oxides of the compounds and N-oxides of the salts thereof may contain, e.g. when isolated in crystalline form, varying amounts of solvents. Included within the scope of the invention are, therefore, all solvates of the compounds of formula (I), the salts thereof, the N-oxides of the compounds and the salts thereof and the stereoisomers of the compounds, salts, N-oxides of the compounds and N-oxides of the salts thereof. Hydrates are a preferred example of said solvates.

The N-oxides of the compounds according to the invention, the salts thereof, the stereoisomers of the compounds and the salts thereof include compounds, wherein the nitrogen atom of the pyridine moiety is oxidized, as illustrated by formula (Ia) below:

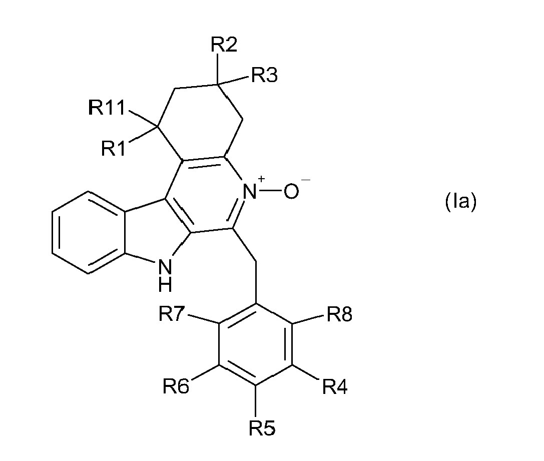

The compounds according to the invention, the salts thereof, the N-oxides of the compounds and the salts thereof include stereoisomers. In case R1 being a hydroxy group, R11 being hydrogen and R2 and R3 representing identical groups, the compounds according to the invention, the salts thereof, the N-oxides of the compounds and the salts thereof have one stereogenic center. In case R1 and R11 being hydrogen or R1 and R11 combining to form an oxo group and R2 and R3 representing different groups, the compounds according to the invention, the salts thereof, the N-oxides of the compounds and the salts thereof have one stereogenic center. In case R1 being a hydroxy group, R11 being hydrogen and R2 and R3 representing different groups, the compounds according to the invention, the salts thereof, the N-oxides of the compounds and the salts thereof have two stereogenic centers. Each of said stereogenic centers may have the absolute configuration R or the absolute configuration S (according to the rules of Cahn, Ingold and Prelog). Accordingly, the stereoisomers (1R), (1S), (3R), (3S), (1R,3R), (1R,3S), (1S,3R) and (1S,3S), wherein the numbers refer to the atoms indicated in formula (lb) below 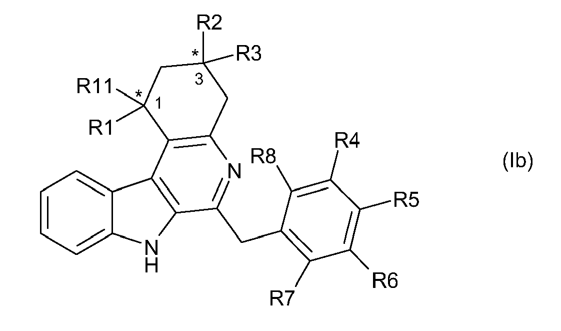 the salts thereof, the N-oxides of the stereoisomers and the salts thereof are part of the invention.

In a preferred embodiment, the invention relates to stereoisomers of formula (lb) having the configuration (1R) or (1S), with the carbon atom at position number 3 not being an asymmetric carbon atom.

The invention further includes all mixtures of the stereoisomers mentioned above independent of the ratio, including the racemates.

Some of the compounds, salts thereof, N-oxides of the compounds and the salts thereof, stereoisomers of the compounds, salts, N-oxides of the compounds and N-oxides of the salts thereof according to the invention may exist in different crystalline forms (polymorphs) which are within the scope of the invention.

Furthermore, derivatives of the compounds of formula (I), the salts thereof, the N-oxides of the compounds or the salts thereof, stereoisomers of the compounds, salts, N-oxides of the compounds or N-oxides of the salts thereof which are converted into compound (I) or a salt thereof, an N-oxide of the compound or the salt thereof, or a stereoisomer of the compound, the salt, the N-oxide of the compound or the N-oxide of the salt thereof in a biological system (bioprecursors or pro-drugs) are covered by the invention. Said biological system is e.g. a mammalian organism, particularly a human subject. The bioprecursor is, for example, converted into the compound of formula (I), a salt thereof, an N-oxide of the compound or the salt thereof, or a stereoisomer of the compound, the salt, the N-oxide of the compound or the N-oxide of the salt thereof by metabolic processes.

The compounds according to the invention can be prepared as follows.

As shown in reaction scheme 1, a compound of formula (1c) wherein R1 and R11 combine to form an oxo group can be obtained by reacting a compound of formula (II) with ammonia in an appropriate solvent, e.g. acetonitrile, preferably under microwave heating. The compound of formula (II) can be prepared by cyclization of a compound of formula (IV) with a compound of formula (III) in the presence of a strong inorganic acid, e.g. perchloric acid, in a suitable solvent, e.g. nitromethane.

Compounds of formula (III) are commercially available or can be obtained according to procedures known in the art.

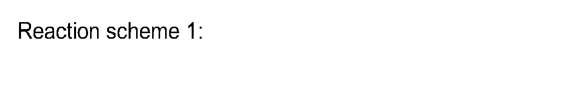

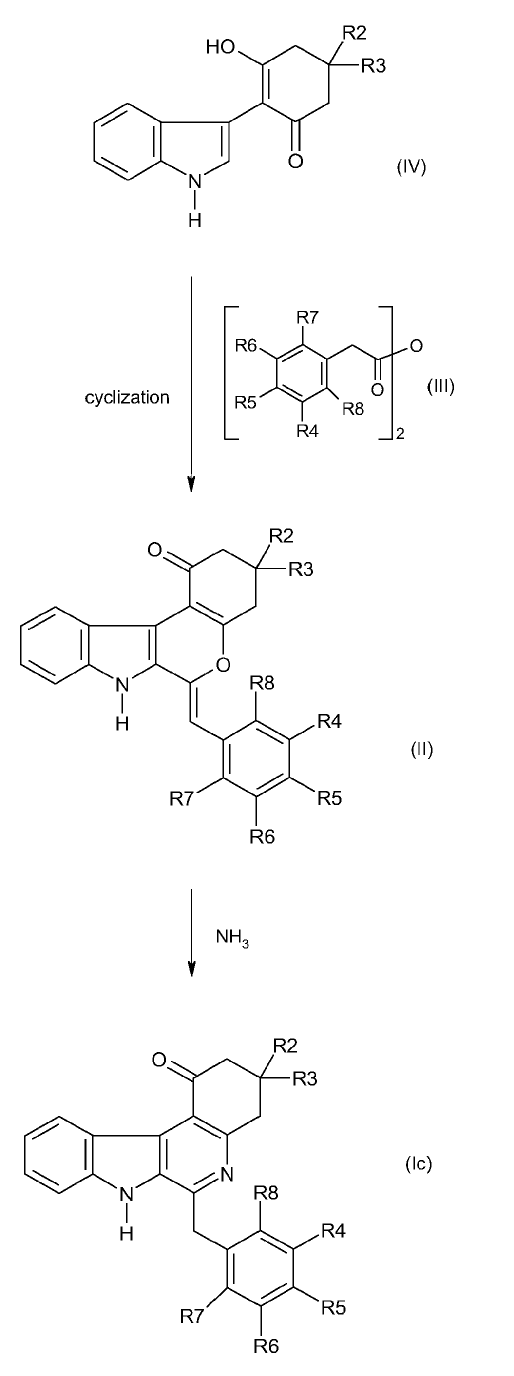

In an alternative procedure, as illustrated in reaction scheme 2, a compound of formula (IV) can be reacted with a compound of formula (VI), in which X is a suitable leaving group, e.g. halogen, such as chlorine, or a conjugate base of an acid, such as trifluoroacetate, in a Friedel-Crafts acylation reaction in the presence of an appropriate Lewis acid, e.g. zinc chloride, boron trifluoride etherate or orthophosphoric acid, in a suitable solvent, e.g. dichloromethane, dichloroethane, diethylether, toluene and/or chlorobenzene, to give the corresponding compound of formula (II) or (V) or a mixture thereof. Said Friedel-Crafts acylation reaction can be based on, for example, Duval et al. (2004) Tetrahedron Letters 45: 5411-5413. The compound of formula (II) or (V) or the mixture thereof can be subjected to a cyclization condensation reaction with ammonium acetate in an appropriate solvent, e.g. acetic acid, preferably at elevated temperature, or a cyclization condensation reaction with ammonia in an appropriate solvent, e.g. methanol, preferably at elevated temperature, to give a corresponding compound of formula (Ic). In some cases it may be convenient to perform both the Friedel-Crafts acylation reaction and the cyclization condensation reaction in one pot.

Compounds of formula (VI) are commercially available or can be obtained according to procedures known in the art.

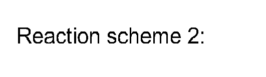

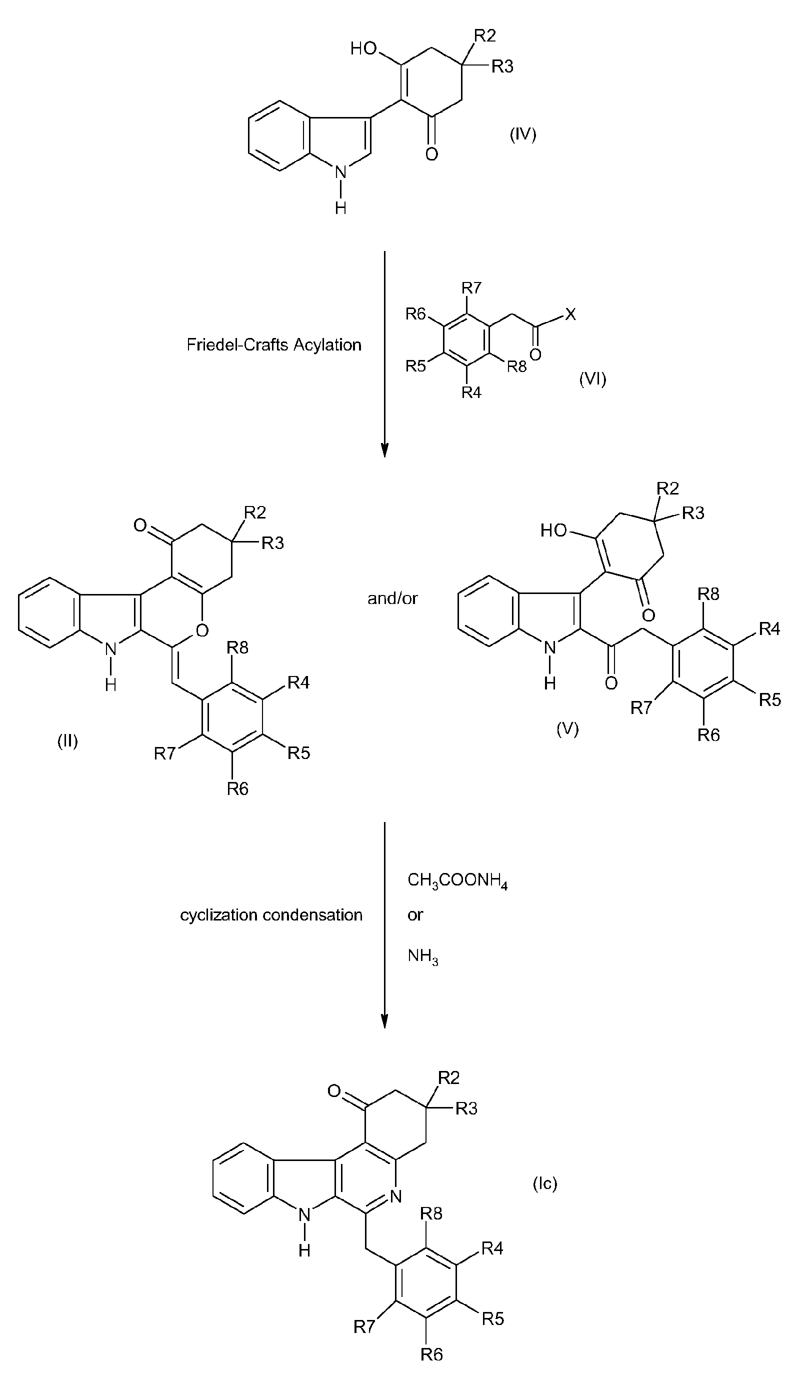

As shown in reaction scheme 3, compounds of formula (IV) are obtainable via an aldol-type condensation of compounds of formula (VIII), in which PG stands for a suitable temporary protecting group, e.g. acetyl, with compounds of formula (VII), and subsequent removal of PG.

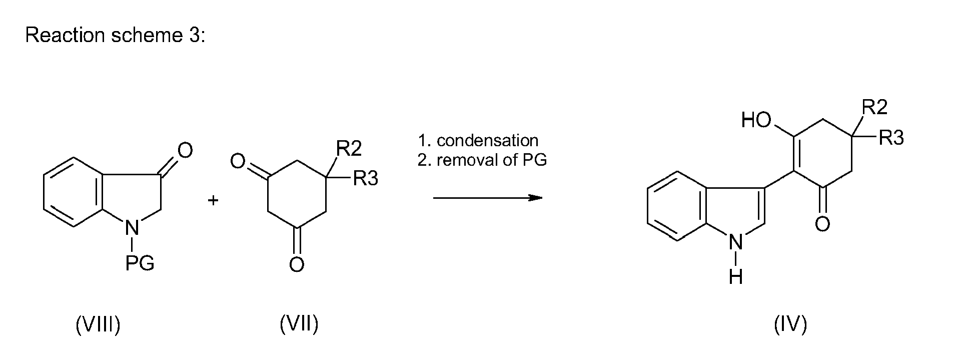

Compounds of formulae (VIII) and (VII) are commercially available or can be obtained according to procedures known in the art.

Furthermore, a compound of formula (Id), in which R1 and R11 are hydrogen, can be obtained as shown in reaction scheme 4. In particular, a compound of formula (XI) can be reacted with a compound of formula (X) in an art-known nucleophilic substitution reaction [see e.g. Heterocycles 31 (8), 1497-1504 (1990)]. The thus obtained hydroxy-compound can be oxidized in a manner known to the skilled person, e.g. according to a Swern oxidation [see e.g. Tetrahedron 47 (41) 8653-8662] to give the corresponding compound of formula (IX). The compound of formula (IX) can be reacted according to reaction scheme 1 or 2 [replacing compound (IV)] to give a compound of formula (Id). The procedure as shown in reaction scheme 4 is preferably used in synthesizing compounds of formula (Id) wherein each of R1, R11, R2 and R3 represents hydrogen.

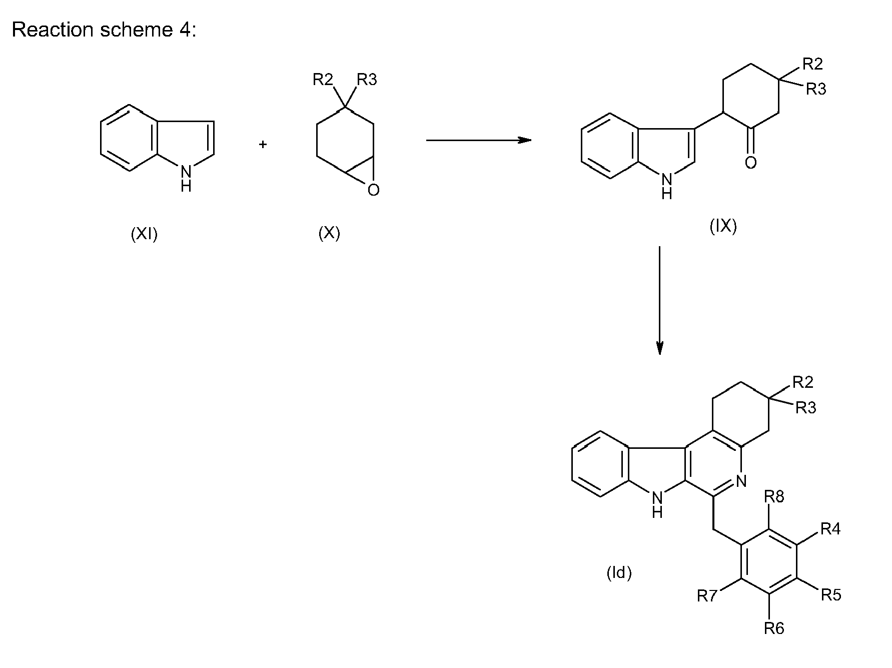

The compounds of formulae (X) and (XI) are known, commercially available or can be obtained according to known procedures.

In particular, a compound of formula (le), in which R1, R11, R2 and R3 are hydrogen, can also be obtained by a condensation reaction as shown in reaction scheme 5. A compound of formula (XI) is, for example, reacted with a compound of formula (XIII) in the presence of acetic acid and H₃PO₄, preferably at elevated temperature. Compound (XIV) thus obtained can be reacted according to reaction scheme 1 or 2 [replacing compound (IV)] to give a compound of formula (le).

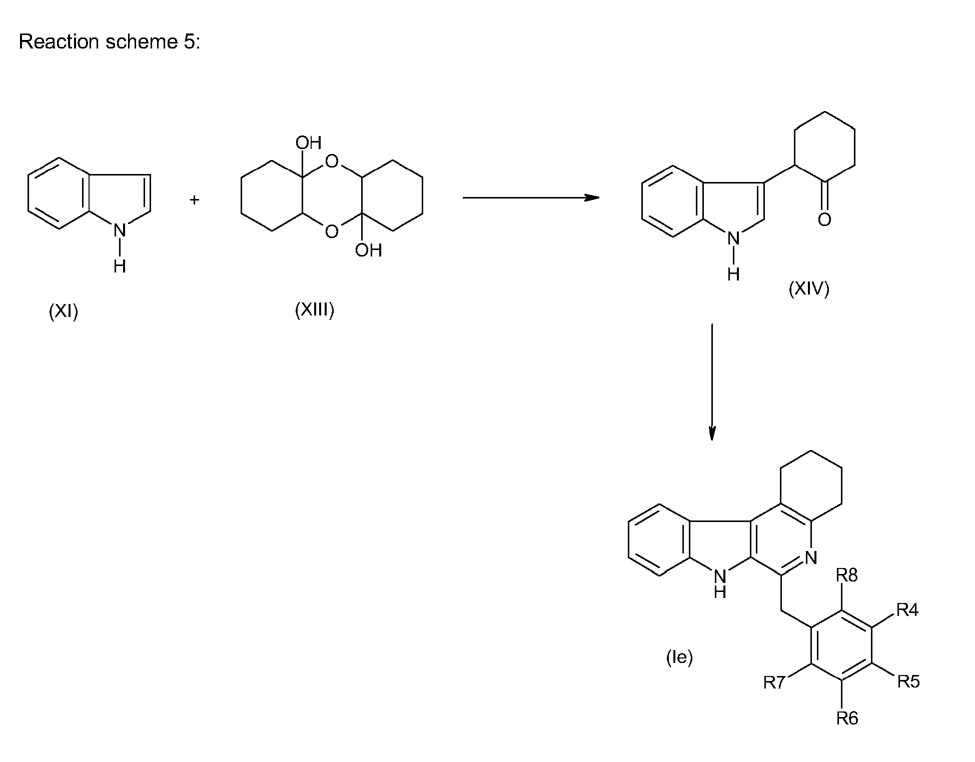

The compounds of formulae (XI) and (XIII) are known, commercially available or can be obtained according to known procedures.

Alternatively, a compound of formula (Id), in which R1 and R11 are hydrogen can be obtained as illustrated in reaction scheme 6. In a first step, a compound of formula (XI) is reacted with a compound of formula (XII) in an art-known radicalic substitution reaction [see e.g. JACS 126, 7450 (2004)]. The thus obtained compound of formula (IX) can be reacted according to reaction scheme 1 or 2 [replacing compound (IV)] to give a compound of formula (Id). The method shown in reaction scheme 6 is preferably used in preparing compounds of formula (Id) in which each of R1, R11 and R2 is hydrogen and R3 is 1-3C-alkyl.

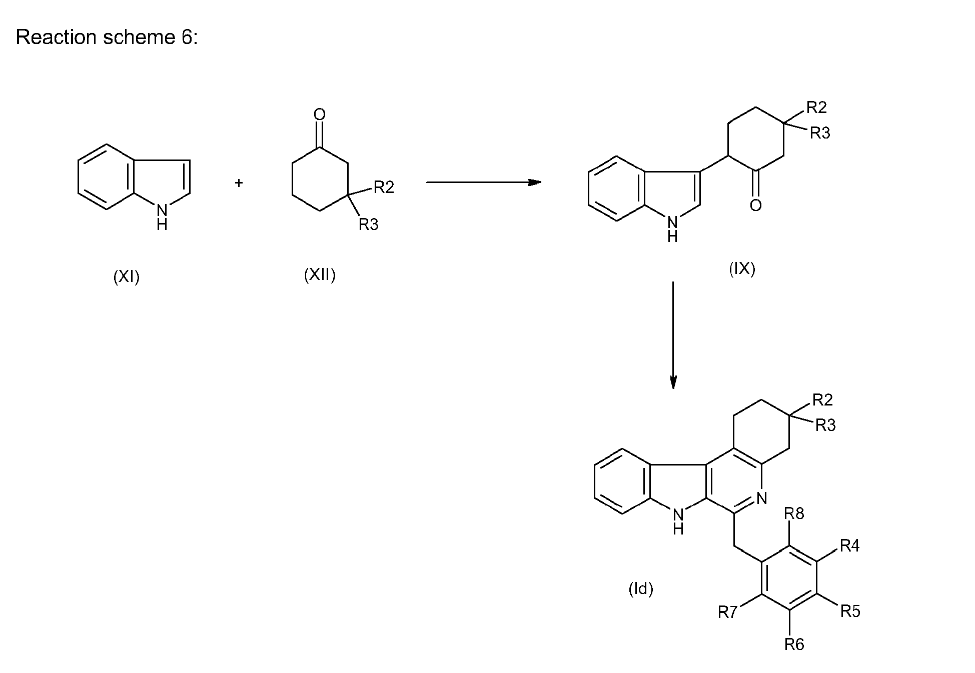

Compounds of formulae (XII) are known, commercially available or can be obtained according to known procedures.

Compounds of formula (I) can be converted into different compounds of formula (I) by methods known in the art. For example,
· a compound of formula (I), wherein R1 is hydroxy and R11 is hydrogen, can be prepared from a compound of formula (I), wherein R1 and R11 combine to form an oxo group, by reduction reaction, e.g. with the aid of a suitable reduction agent, such as sodium borohydride;
· a compound of formula (I), wherein R1 and R11 are hydrogen, can be prepared from a compound of formula (I), wherein R1 and R11 combine to form an oxo group, by reduction reaction, e.g. with the aid of a suitable reduction agent, such as hydrazine (e.g. according to a Wolff-Kishner reduction);
· a compound of formula (I), wherein R4 and/or R5 represent(s) a nitro group can be converted into the corresponding amino compound by reduction reaction, e.g. with the aid of a suitable reduction agent, such as tin dichloride or hydrogen gas and a palladium on carbon catalyst;
· a compound of formula (I), wherein R5 represents a group -NH-C(O)-1-2C-alkyl can be prepared e.g. from a compound of formula (I), wherein R5 represents an amino group by reaction with an appropriate carboxylic acid chloride or carboxylic anhydride, in the presence of a base, e.g. triethylamine, pyridine or potassium carbonate, or with an appropriate carboxylic acid in the presence of a dehydrating agent, e.g. dicyclohexylcarbodiimide;
· a compound of formula (I), wherein R5 represents -NH-C(O)-NH₂ can be obtained e.g. from a compound of formula (I), wherein R5 represents an amino group by reaction with potassium cyanate in the presence of a mineral acid, such as hydrochloric acid, or by condensation with urea;
· a compound of formula (I), wherein R5 is hydroxy can be synthesized e.g. from a compound of formula (I), wherein R5 is 1-3C-alkoxy by dealkylation with a Lewis acid, such as boron tribromide.

It is known to the person skilled in the art that, if there are a number of reactive centers on a starting or intermediate compound, it may be necessary to block one or more reactive centers temporarily by protective groups in order to allow a reaction to proceed specifically at the desired reaction center.

The compounds according to the invention are isolated and purified in a manner known per se, e.g. by distilling off the solvent in vacuo and recrystallizing the residue obtained from a suitable solvent or subjecting it to one of the customary purification methods, such as column chromatography on a suitable support material.

Salts of the compounds of formula (I), the N-oxides thereof and the stereoisomers of the compounds and the N-oxides thereof according to the invention can be obtained by dissolving the free compound in a suitable solvent (for example a ketone such as acetone, methylethylketone or methylisobutylketone, an ether such as diethyl ether, tetrahydrofurane or dioxane, a chlorinated hydrocarbon such as methylene chloride or chloroform, a low molecular weight aliphatic alcohol such as methanol, ethanol or isopropanol, a low molecular weight aliphatic ester such as ethyl acetate or isopropyl acetate, or water) which contains the desired acid or base, or to which the desired acid or base is then added. The acid or base can be employed in salt preparation, depending on whether a mono- or polybasic acid or base is concerned and depending on which salt is desired, in an equimolar quantitative ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art.

The compounds of formula (I), the salts thereof and the stereoisomers of the compounds and the salts according to the invention can be converted into their N-oxides, for example, by reaction with peracids, such as m-chloroperbenzoic acid or peracetic acid. The person skilled in the art is familiar with the reaction conditions for carrying out the N-oxidation.

Pure diastereomers and pure enantiomers of the compounds of formula (I), the salts thereof, the N-oxides of the compounds and the N-oxides of the salts according to the invention can be obtained e.g. by asymmetric synthesis, by using chiral starting compounds in synthesis and/or by splitting up enantiomeric and diasteriomeric mixtures obtained in synthesis. Preferably, the pure diastereomeric and pure enantiomeric compounds of the invention are obtainable by asymmetric synthesis and/or by using chiral starting compounds in synthesis.

In particular, for example the (1S)-enantiomers of the compounds of formula (I), the salts thereof, the N-oxides of the compounds and the salts thereof according to the invention can be obtained by reduction of the corresponding ketone precursors (wherein R1 and R11 combine to form an oxo group) with sodium borohydride in the presence of (4S,5S)-2-(3-nitro-phenyl)-[1,3,2]dioxaborolane-4,5-dicarboxylic acid, which can be prepared by esterification of 3-nitrophenyl boronic acid and D-tartaric acid in the presence of a dehydrating agent such as calcium hydride. Likewise, for example the (1R)-enantiomers of the compounds of formula (I), the salts thereof, the N-oxides of the compounds and the salts thereof according to the invention can be obtained using (4R,5R)-2-(3-nitro-phenyl)-[1,3,2]dioxaborolane-4,5-dicarboxylic acid, which can be prepared by esterification of 3-nitrophenyl boronic acid and L-tartaric acid in the presence of a dehydrating agent such as calcium hydride.

Enantiomeric and diastereomeric mixtures can be split up into the pure enantiomers and pure diastereomers by methods known to a person skilled in the art. Preferably, diastereomeric mixtures are separated by crystallization, in particular fractional crystallization, or chromatography. Enantiomeric mixtures can be separated e.g. by forming diastereomers with a chiral auxiliary agent, resolving the diastereomers obtained and removing the chiral auxiliary agent. As chiral auxiliary agents, for example, chiral acids can be used to separate enantiomeric bases and chiral bases can be used to separate enantiomeric acids via formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be split up using chiral separating columns in chromatography. Another suitable method for the isolation of enantiomers is the enzymatic separation.

As will be appreciated by persons skilled in the art, the invention is not limited to the particular embodiments described herein, but covers all modifications of said embodiments that are within the spirit and scope of the invention as defined by the appended claims.

All patents, patent applications, publications, test methods and other materials cited herein are incorporated by reference in their entireties.

The following examples illustrate the invention in greater detail, without restricting it. Further compounds according to the invention, of which the preparation is not explicitly described, can be prepared in an analogous way.

The compounds which are mentioned in the examples, the salts thereof, N-oxides of the compounds and the salts thereof and stereoisomers of the compounds, salts, N-oxides of the compounds and N-oxides of the salts thereof represent preferred embodiments of the invention.

### Examples

The following abbreviations are used: min: minutes, h: hour(s), DCM: dichloromethane, DCE: dichloroethane, THF: tetrahydrofurane, EA: ethyl acetate, mp.: melting point, RT: room temperature (20 to 25°C), TLC: thin layer chromatography, MS: mass spectrometry, ¹H-NMR: ¹H nuclear magnetic resonance spectroscopy.

### Final Compounds

### 1. 6-(4-Methoxy-benzyl)-3,3-dimethyl-2,3,4,7-tetrahydro-indolo[2,3-c]quinolin-1-one

Step 1: 5.08 g of 3-hydroxy-2-(1 H-indol-3-yl)-5,5-dimethyl-cyclohex-2-enone (compound A1) and 62.55 g of 4-methoxyphenyl acetic acid anhydride are solved in 150 ml nitromethane. The resulting solution is treated 6 times (every 10 minutes) with 0.2 ml of a 70% (v/v) aqueous HClO₄ solution and stirred for one additional hour. The reaction mixture is diluted with 100 ml dichloromethane and 50 ml saturated aqueous NaCO₃ solution. The organic layer is separated and the aqueous layer is extracted again. The combined organic extracts are dried and crystallized from nitromethane to give 4.65 g 6-(4-methoxy-benzylidene)-3,3-dimethyl-3,4,6,7-tetrahydro-2H-5-oxa-7-aza-benzo[c]fluoren-1-one.
¹H-NMR (200 MHz, CDCl₃): δ = 1.17 (s,6H), 2.46 (s,2H), 2.63 (s,2H), 3.85 (s,3H), 6.0 (s,1H), 6.92-6.96 (m,2H), 7.14-7.32 (m,3H), 7.56-7.62 (m,2H), 8.07 (br,1H), 8.81 (d,J=7.5Hz,1H)
Step 2: 200 mg of 6-(4-methoxy-benzylidene)-3,3-dimethyl-3,4,6,7-tetrahydro-2H-5-oxa-7-aza-benzo[c]fluoren-1-one is suspended in 10 ml acetonitrile and treated with 10 ml 25% (w/v) aqueous NH₃ solution. The reaction mixture is heated in a sealed vial by microwave heating (130°C) for 25 min. The solvent is removed and the residue is dissolved in dichloromethane and water. After separation of the organic layer, the aqueous layer is extracted with dichloromethane, the combined organic layers are dried and evaporated to dryness. The residue is purified by flash chromatography to give 134 mg of the title compound.
¹H-NMR (200 MHz, CDCl₃): δ= 1.19 (s,6H), 2.71 (s,2H), 3.24 (s,2H), 3.77 (s,3H), 4.50 (s,2H), 6.82-6.88 (m,2H), 7.19-7.34 (m,4H), 7.99 (br,1H), 9.34 (d,J=8.3Hz,1H)
mp.: 169°C

### Alternative procedure:

Step 1: 10 g p-methoxyphenylacetylchloride is dissolved in 200 ml dry DCM. To this solution is added 7.4 g ZnCl₂ and 4.6 g 3-hydroxy-2-(1H-indol-3-yl)-5,5-dimethyl-cyclohex-2-enone (compound A1). 25 ml of diethyl ether are added to dissolve the occurred slurry, followed by stirring for 2 h at room temperature. The reaction mixture is poured into ice-cold 1 M HCl solution and the product is extracted with DCM. The organic layer is washed with 2 x 25 ml water and 2 x 25 ml saturated sodium bicarbonate solution. The organic layer is dried over MgSO₄, filtered and evaporated. The crude 3-hydroxy-2-{2-[2-(4-methoxy-phenyl)-ethanoyl]-1 H-indol-3-yl}-5,5-dimethyl-cyclohex-2-enone thus obtained is used immediately without further purification in the next step. Step 2: The crude 3-hydroxy-2-{2-[2-(4-methoxy-phenyl)-ethanoyl]-1 H-indol-3-yl}-5,5-dimethyl-cyclohex-2-enone is dissolved in 100 ml acetic acid and 27.8 g ammonium acetate is added. The mixture is stirred for 2 h at 90°C. The acetic acid is evaporated. 100 ml ethyl acetate and 200 ml saturated sodium bicarbonate solution are added and separated. The organic layer is washed with 100 ml saturated sodium bicarbonate solution, dried with MgSO₄ and evaporated. The product is purified by chromatography with an eluens system of petroleum ether / ethyl acetate (2/1 v/v) with 2% (v/v) triethylamine. The obtained oil is crystallized from a diethyl ether / HCl solution to yield 3.6 g of the HCl salt.

The following compounds are obtained by using the first described procedure of Example 1 analogously.

### 2. 6-(4-Methoxy-benzyl)-2,3,4,7-tetrahydro-indolo[2,3-c]quinolin-1-one

Starting compounds: 3-Hydroxy-2-(1H-indol-3-yl)-cyclohex-2-enone (compound A3) and 4-methoxyphenyl acetic acid anhydride
¹H-NMR (200 MHz, CDCl₃): δ = 2.20-2.33 (m,2H), 2.86 (t,J=6.1 Hz,2H), 3.38 (t,J=6.2Hz,2H), 3.74 (s,3H), 4.57 (s,2H), 6.77-6.83 (m,2H), 7.23-7.32 (m,3H), 7.38-7.42 (m,1H), 7.50-7.61 (m,1H), 8.52 (br,1H), 9.31 (d,J=8.3Hz,1H)
mp.: 177°C

### 3. 6-Benzyl-2,3,4,7-tetrahydro-indolo[2,3-c]quinolin-1-one

Starting compounds: 3-Hydroxy-2-(1H-indol-3-yl)-cyclohex-2-enone (compound A3) and phenyl acetic acid anhydride
¹H-NMR (200 MHz, D₆-DMSO): δ = 2.10-2.16 (m,2H), 2.77 (t,J=6.1 Hz,2H), 3.19 (t,J=6.2Hz,2H), 4.52 (s,2H), 7.13-7.31 (m,4H), 7.35-7.39 (m,2H), 7.54-7.66 (m,2h), 9.21 (d,J=8.4Hz,1H), 12.0 (br,1 H)
mp.: 205°C

### 4. 6-(4-Methoxy-benzyl)-3-methyl-2,3,4,7-tetrahydro-1 H-indolo[2,3-c]quinoline

Starting compounds: 2-(1H-Indol-3-yl)-5-methyl-cyclohexanone (compound A5) and 4-methoxyphenyl acetic acid anhydride
¹H-NMR (400 MHz, d₆-DMSO): δ= 1.10 (d,J=6.5Hz,3H), 1.45-1.55 (m,1H), 1.97-2.07 (m,2H), 2.54-2.61 (m,1H), 3.02 (dd,J=16.4,J=4Hz,1H), 3.15-3.23 (m,1H), 3.36-3.40 (m,1H), 3.66 (s,3H), 4.35 (s,2H), 6.81 (d,J=8.7Hz,2H), 7.19-7.23 (m,1H), 7.28 (d,J=8.7Hz,2H), 7.50-7.54 (m,1H), 7.60 (d,J=8.2Hz,1H),8.14 (d, J=8Hz, 1H), 11.60 (br,1H)
mp.: 186°C

The following compounds are obtained by using the second described procedure of Example 1 analogously.

### 5. 6-(4-Bromo-benzyl)-3,3-dimethyl-2,3,4,7-tetrahydro-indolo[2,3-c]quinolin-1-one, HCl salt

Starting compounds: 4-bromo-phenylacetylchloride and 3-hydroxy-2-(1H-indol-3-yl)- 5,5-dimethyl-cyclohex-2-enone (compound A1)
¹H-NMR (200 MHz, CDCl₃): δ= 1.16 (s, 6H), 2.71 (s, 2H), 3.51 (s, 2H), 5.16 (s, 2H), 6.95-6.99 (m, 2H), 7.32-7.40 (m, 1H), 7.63-7.75 (m, 3H), 7.99 (d, J=8.3 Hz,1H), 9.26 (d, J=8.5 Hz,1H), 13.27 (bs, 1H,), 15.33 (bs, 1H)
mp.: 244-245°C

### 6. 6-(4-Methoxy-benzyl)-2,3,4,7-tetrahydro-1H-indolo[2,3-c]quinoline, HCl salt

Starting compounds: 4-methoxy-phenylacetylchloride and 2-(1H-indol-3-yl)-cyclohexanone (compound A4)
mp.: decomposes at 231°C

### 7. 6-Benzo[1,3]dioxol-5-ylmethyl-3,3-dimethyl-2,3,4,7-tetrahydro-indolo[2,3-c]quinolin-1-one, HCl salt

Starting compounds: Benzo[1,3]dioxol-5-yl-acetylchloride and 3-hydroxy-2-(1H-indol-3-yl)- 5,5-dimethyl-cyclohex-2-enone (compound A1)
mp.: decomposes at 200°C

### 8. 3,3-Dimethyl-6-(4-nitro-benzyl)-2,3,4,7-tetrahydro-indolo[2,3-c]quinolin-1-one, HCl salt

Starting compounds: 4-nitro-phenylacetylchloride and 3-hydroxy-2-(1H-indol-3-yl)- 5,5-dimethyl-cyclohex-2-enone (compound A1)
mp.:214-215°C

### 9. 6-(4-Bromo-benzyl)-2,3,4,7-tetrahydro-1H-indolo[2,3-c]quinoline, HCl salt

Starting compounds: 4-bromo-phenylacetylchloride and 2-(1H-indol-3-yl)-cyclohexanone (compound A4)
mp.: 311-312°C

### 10. 6-(3-Methoxy-benzyl)-3,3-dimethyl-2,3,4,7-tetrahydro-indolo[2,3-c]quinolin-1-one

In a 20 ml microwave vial, 0.57 g 3-hydroxy-2-(1 H-indol-3-yl)- 5,5-dimethyl-cyclohex-2-enone (compound A1) is suspended in 2 ml DCE and 5 ml of a 1M ZnCl₂ solution in ether is added. To this solution, 0.92 g 3-methoxy-phenylacetylchloride dissolved in 2 ml dry DCE is added and stirred for 2 h at room temperature. Slowly, 5 ml of a 7N NH₃ solution in methanol is added and the mixture is heated in a Biotage Initiator microwave reactor for 30 min at 150°C. The mixture is diluted with 25 ml ethyl acetate and 20 ml 2M aqueous NH₃. The organic layer is washed with brine (saturated sodium chloride solution), dried over MgSO₄, filtered and concentrated in vacuo. The product is purified by chromatography [Silicagel, petroleum ether / ethyl acetate (3/1 v/v)] and crystallized from ethyl acetate / diethyl ether to yield 0.37 g of the title compound.
¹H-NMR (200 MHz, CDCl3): δ = 1.17 (s,6H), 2.76 (s,2H), 3.26 (s,2H), 3.72 (s,3H), 4.55 (s,2H), 6.70-6.95 (m,3H), 7.15-7.40 (m,3H), 7.51 (t,J=7.0 Hz,1H), 8.10 (brs,1H), 9.31 (d,J=8.4Hz,1H) MS (MH⁺ found) = 385.3

The following compounds are obtained by using the procedure of Example 10 analogously:

### 11. 3,3-Dimethyl-6-(4-trifluoromethoxy-benzyl)-2,3,4,7-tetrahydro-indolo[2,3-c]quinolin-1-one

Starting compounds: 4-Trifluoromethoxy-phenylacetylchloride and 3-hydroxy-2-(1H-indol-3-yl)- 5,5-dimethyl-cyclohex-2-enone (compound A1)
mp.: 141-143°C
MS (MH⁺ found) = 439.4

### 12. 6-(4-Ethoxy-benzyl)-3,3-dimethyl-2,3,4,7-tetrahydro-indolo[2,3-c]quinolin-1-one

Starting compounds: 4-Ethoxy-phenylacetylchloride and 3-hydroxy-2-(1H-indol-3-yl)- 5,5-dimethyl-cyclohex-2-enone (compound A1)
mp.: 179-181°C
MS (MH⁺ found) = 399.4

### 13. 3,3-Dimethyl-6-(3-nitro-benzyl)-2,3,4,7-tetrahydro-indolo[2,3-c]quinolin-1-one

Starting compounds: 3-Nitro-phenylacetylchloride and 3-hydroxy-2-(1H-indol-3-yl)- 5,5-dimethyl-cyclohex-2-enone (compound A1)
mp.: decomposes at 236°C
MS (MH⁺ found) = 400.3

### 14. 6-(4-Isopropyl-benzyl)-3,3-dimethyl-2,3,4,7-tetrahydro-indolo[2,3-c]quinolin-1-one

Starting compounds: 4-Isopropyl-phenylacetylchloride and 3-hydroxy-2-(1H-indol-3-yl)-5,5-dimethyl-cyclohex-2-enone (compound A1)
mp.: 153-155°C
MS (MH⁺ found) = 397.4

### 15. 6-(3-Chloro-4-methoxy-benzyl)-3,3-dimethyl-2,3,4,7-tetrahydro-indolo[2,3-c]quinolin-1-one

Starting compounds: 3-Chloro-4-methoxy-phenylacetylchloride and 3-hydroxy-2-(1H-indol-3-yl)-5,5-dimethyl-cyclohex-2-enone (compound A1)
mp.: 209-211 °C
MS (MH⁺ found) = 419.3

### 16. 3,3-Dimethyl-6-(4-nitro-benzyl)-2,3,4,7-tetrahydro-indolo[2,3-c]quinolin-1-one

Starting compounds: 4-Nitro-phenylacetylchloride and 3-hydroxy-2-(1H-indol-3-yl)- 5,5-dimethyl-cyclohex-2-enone (compound A1)
mp.: 164-166°C

### 17. 6-(4-Methoxy-benzyl)-3-methyl-2,3,4,7-tetrahydro-indolo[2,3-c]quinolin-1-one

Starting compounds: 4-Methoxy-phenylacetylchloride and 3-hydroxy-2-(1H-indol-3-yl)- 5-methyl-cyclohex-2-enone (compound A2)
mp.: 102°C
MS (MH⁺ found) = 371.3

### 18. 6-Benzo[1,3]dioxol-5-ylmethyl-2,3,4,7-tetrahydro-indolo[2,3-c]quinolin-1-one

Starting compounds: Benzo[1,3]dioxol-5-yl-acetyl chloride and 3-hydroxy-2-(1H-indol-3-yl)-cyclohex-2-enone (compound A3)
mp.: 194-197°C
MS (MH⁺ found) = 371.3

### 19. 6-(3-Nitro-benzyl)-2,3,4,7-tetrahydro-indolo[2,3-c]quinolin-1-one

Starting compounds: 3-Nitro-phenylacetylchloride and 3-hydroxy-2-(1H-indol-3-yl)-cyclohex-2-enone (compound A3)
mp.: decomposes at 221°C
MS (MH⁺ found) = 372.3

### 20. 6-(4-Chloro-benzyl)-3,3-dimethyl-2,3,4,7-tetrahydro-indolo[2,3-c]quinolin-1-one

1.36 g 4-chlorophenylacetic acid is stirred with 1.1 ml trifluoroacetic anhydride at RT, after 15 min the solution is diluted with 3 ml DCE and added to a cooled solution of 1.02 g 3-hydroxy-2-(1H-indol-3-yl)-5,5-dimethyl-cyclohex-2-enone (compound A1) in 3 ml DCE and 8 ml of a 1M ZnCl₂ solution in ether. The mixture is stirred at RT for 1 h. 6 ml of a 7N NH₃ solution in methanol is added slowly and the mixture is heated at 80°C for 17 h. The mixture is cooled to RT and 25 ml EA and 20 ml 2M ammonia are added. The organic layer is separated, dried with MgSO₄ and concentrated in vacuo. The product is purified by chromatography [Silicagel, petroleum ether / ethyl acetate (3/1 v/v)] and crystallized from ethyl acetate / diethyl ether to yield 0.45 g of the title compound.
mp.: 205-207°C
MS (MH⁺ found) = 389.3

The following compounds are obtained by using the procedure of Example 20 analogously:

### 21. 6-(3-Bromo-benzyl)-3,3-dimethyl-2,3,4,7-tetrahydro-indolo[2,3-c]quinolin-1-one

Starting compounds: 3-Bromophenylacetic acid and 3-hydroxy-2-(1H-indol-3-yl)-5,5-dimethyl-cyclohex-2-enone (compound A1)
m p.: 215-219°C
MS (MH⁺ found) = 433.3 and 435.3

### 22. 6-(3,5-Difluoro-benzyl)-3,3-dimethyl-2,3,4,7-tetrahydro-indolo[2,3-c]quinolin-1-one

Starting compounds: 3,5-Difluorophenylacetic acid and 3-hydroxy-2-(1H-indol-3-yl)-5,5-dimethyl-cyclohex-2-enone (compound A1)
mp.: 204-208°C
MS (MH⁺ found) = 391.3

### 23. 6-(3-Fluoro-4-methoxy-benzyl)-3,3-dimethyl-2,3,4,7-tetrahydro-indolo[2,3-c]quinolin-1-one

Starting compounds: 3-Fluoro-4-methoxy-phenylacetic acid and 3-hydroxy-2-(1H-indol-3-yl)-5,5-dimethyl-cyclohex-2-enone (compound A1)
mp.: 192-195°C
MS (MH⁺ found) = 403.3

### 24. 6-(3,4-Difluoro-benzyl)-3,3-dimethyl-2,3,4,7-tetrahydro-indolo[2,3-c]quinolin-1-one

Starting compounds: 3,4-Difluorophenylacetic acid and 3-hydroxy-2-(1H-indol-3-yl)-5,5-dimethyl-cyclohex-2-enone (compound A1)
mp.: 204-207°C
MS (MH⁺ found) = 391.3

### 25. 3,3-Dimethyl-6-(4-methyl-benzyl)-2,3,4,7-tetrahydro-indolo[2,3-c]quinolin-1-one

Starting compounds: 4-Methylphenylacetic acid and 3-hydroxy-2-(1H-indol-3-yl)-5,5-dimethyl-cyclohex-2-enone (compound A1)
mp.: 182-184°C
MS (MH⁺ found) = 369.4

### 26. 6-(4-Nitro-benzyl)-2,3,4,7-tetrahydro-indolo[2,3-c]quinolin-1-one

Starting compounds: 4-Nitrophenylacetic acid and 3-hydroxy-2-(1H-indol-3-yl)-cyclohex-2-enone (compound A3)
mp.: 138-140°C
MS (MH⁺ found) = 372.2

### 27. 6-(2-Fluoro-4-methoxy-benzyl)-3,3-dimethyl-2,3,4,7-tetrahydro-indolo[2,3-c]quinolin-1-one

Starting compounds: 2-Fluoro-4-methoxy-phenylacetic acid and 3-hydroxy-2-(1H-indol-3-yl)-5,5-dimethyl-cyclohex-2-enone (compound A1)
mp.: 138-140°C
MS (MH⁺ found) = 403.3

### 28. 6-(4-Fluoro-benzyl)-3,3-dimethyl-2,3,4,7-tetrahydro-indolo[2,3-c]quinolin-1-one

Starting compounds: 4-Fluorophenylacetic acid and 3-hydroxy-2-(1H-indol-3-yl)-5,5-dimethyl-cyclohex-2-enone (compound A1)
mp.: 200-204°C
MS (MH⁺ found) = 373.4

### 29. 6-(3-Fluoro-4-methoxy-benzyl)-2,3,4,7-tetrahydro-indolo[2,3-c]quinolin-1-one

Starting compounds: 3-Fluoro-4-methoxy-phenylacetic acid and 3-hydroxy-2-(1H-indol-3-yl)-cyclohex-2-enone (compound A3)
mp.: 228-230°C
MS (MH⁺ found) = 375.3

### 30. 6-(3-Fluoro-4-methoxy-benzyl)-2,3,4,7-tetrahydro-1 H-indolo[2,3-c]quinoline

Starting compounds: 3-Fluoro-4-methoxy-phenylacetic acid and 2-(1H-indol-3-yl)-cyclohexanone (compound A4)
mp.: 118-120°C
MS (MH⁺ found) = 361.3

### 31. 6-(2,3-Difluoro-4-methoxy-benzyl)-3,3-dimethyl-2,3,4,7-tetrahydro-indolo[2,3-c]quinolin-1-one

Starting compounds: 2,3-Difluoro-4-methoxy-phenylacetic acid and 3-hydroxy-2-(1H-indol-3-yl)-5,5-dimethyl-cyclohex-2-enone (compound A1)
mp.: 199-200°C
MS (MH⁺ found) = 421.4

### 32. 6-(3,5-Difluoro-4-methoxy-benzyl)-3,3-dimethyl-2,3,4,7-tetrahydro-indolo[2,3-c]quinolin-1-one

Starting compounds: 3,5-Difluoro-4-methoxy-phenylacetic acid and 3-hydroxy-2-(1H-indol-3-yl)-5,5-dimethyl-cyclohex-2-enone (compound A1)
mp.: 195-196°C
MS (MH⁺ found) = 421.4

### 33. 6-(2,3-Dihydro-benzofuran-5-ylmethyl)-3,3-dimethyl-2,3,4,7-tetrahydro-indolo[2,3-c]quinolin-1-one

Starting compound: (2,3-Dihydro-benzofuran-5-yl)-acetic acid and 3-hydroxy-2-(1H-indol-3-yl)-5,5-dimethyl-cyclohex-2-enone (compound A1)

### 34. 6-(3-Fluoro-4-hydroxy-benzyl)-3,3-dimethyl-2,3,4,7-tetrahydro-indolo[2,3-c]quinolin-1-one

0.80 g 6-(3-fluoro-4-methoxy-benzyl)-3,3-dimethyl-2,3,4,7-tetrahydro-indolo[2,3-c]quinolin-1-one (compound 23) is dissolved in 10 ml DCM and cooled in an ice bath. 8 ml of a 1 M solution of BBr₃ in DCM is added and stirred for 1 h. 25 ml ice water is added and the mixture is extracted with 75 ml ethyl acetate. The organic layer is washed with brine, dried with MgSO₄ and concentrated in vacuo. The product is triturated with diethyl ether to yield 0.59 g of the title compound.
mp.: 216-218°C
MS (MH⁺ found) = 389.4

### 35. 6-(4-Hydroxy-benzyl)-3,3-dimethyl-2,3,4,7-tetrahydro-indolo[2,3-c]quinolin-1-one

100 mg 6-(4-methoxy-benzyl)-3,3-dimethyl-2,3,4,7-tetrahydro-indolo[2,3-c]quinolin-1-one (compound 1) is solved in dichloromethane and cooled to -78°C. 2.6 ml boro tribromide is added and the reaction mixture is stirred for 10 min at -78°C. Allowing to warm up to room temperature, the mixture is stirred for additional 12 h. 20 ml water and 20 ml dichloromethane are added and the mixture is worked up extractively. The combined organic layers are evaporated and the residue is purified by flash chromatography to give 98 mg of the title compound.
¹H-NMR (400 MHz, d₆-DMSO): δ = 1.10 (s,6H), 2.67 (s,2H), 3.20 (s,2H), 4.49 (s,2H), 6.66 (d, J=8.4Hz,2H), 7.19 (d,J=8.4Hz,2H), 7.28 (br,1H), 7.65-7.72 (m,2H), 9.23 (d,J=8.4Hz,2H), 12.2 (br,1 H)
mp.: decomposes at 210°C

### 36. 6-(4-Amino-benzyl)-3,3-dimethyl-2,3,4,7-tetrahydro-indolo[2,3-c]quinolin-1-one

0.87 g 3,3-Dimethyl-6-(4-nitro-benzyl)- 2,3,4,7-tetrahydro-indolo[2,3-c]quinolin-1-one (compound 16) and 2.2 g SnCl₂. 2H₂O are suspended in 20 ml ethanol and heated at 80°C for 2 h. The mixture is concentrated in vacuo and the residue is stirred with 50 ml DCM and 50 ml 1 M NaOH. The organic layer is separated, dried with MgSO₄ and concentrated in vacuo. The product is crystallized from ethyl acetate / diethyl ether to yield 0.59 g of the title compound.
¹H-NMR (200 MHz, CDCl₃): δ = 1.09 (s, 6H), 2.61 (s, 2H), 3.14 (s, 2H), 3,66 (bs, 2H), 4.37 (bs, 2H), 6.48-6.52 (m, 2H), 6.98-7.02 (m, 2H), 7.12-7.20 (m,1H), 7.33-7.47 (m, 2H), 9.25 (d, J=8.3 Hz, 1H), 9.59 (bs, 1 H)
mp.: 123-124°C

The following compounds are obtained by using the procedure of Example 36 analogously:

### 37. 6-(3-Amino-benzyl)-3,3-dimethyl-2,3,4,7-tetrahydro-indolo[2,3-c]quinolin-1-one

Starting compound: 3,3-Dimethyl-6-(3-nitro-benzyl)-2,3,4,7-tetrahydro-indolo[2,3-c]quinolin-1-one (compound 13)
mp.: 232-234°C
MS (MH⁺ found) = 370.3

### 38. 6-(4-Amino-benzyl)-2,3,4,7-tetrahydro-indolo[2,3-c]quinolin-1-one

Starting compound: 6-(4-Nitro-benzyl)-2,3,4,7-tetrahydro-indolo[2,3-c]quinolin-1-one (compound 26)
mp.: decomposes at 210°C
MS (MH⁺ found) = 342.3

### 39. 6-(3-Amino-benzyl)-2,3,4,7-tetrahydro-indolo[2,3-c]quinolin-1-one

Starting compound: 6-(3-Nitro-benzyl)-2,3,4,7-tetrahydro-indolo[2,3-c]quinolin-1-one (compound 19)
mp.: decomposes at 250°C
MS (MH⁺ found) =342.3

### 40. N-[4-(3,3-Dimethyl-1-oxo-2,3,4,7-tetrahydro-1H-indolo[2,3-c]quinolin-6-ylmethyl)-phenyl]-acetamide

0.4 g 6-(4-amino-benzyl)-3,3-dimethyl-2,3,4,7-tetrahydro-indolo[2,3-c]quinolin-1-one (compound 36) is dissolved in 20 ml DCM and 0.3 ml triethylamine is added followed by 0.10 ml acetyl chloride. The mixture is stirred at RT for 1 h, washed with 1 M NaCO₃ solution, dried with MgSO₄ and concentrated in vacuo. The product is crystallized from ethyl acetate / petroleum ether to yield 0.18 g of the title compound.
mp.: 153-154°C

The following compounds are obtained by using the procedure of Example 40 analogously:

### 41. N-[4-(3,3-Dimethyl-1-oxo-2,3,4,7-tetrahydro-1H-indolo[2,3-c]quinolin-6-ylmethyl)-phenyl]-propionamide

Starting compounds: 6-(4-Amino-benzyl)-3,3-dimethyl-2,3,4,7-tetrahydro-indolo[2,3-c]quinolin-1-one (compound 36) and propionyl chloride
mp.: decomposes at 235°C
MS (MH⁺ found) = 426.4

### 42. N-[4-(1-Oxo-2,3,4,7-tetrahydro-1H-indolo[2,3-c]quinolin-6-ylmethyl)-phenyl]-acetamide

Starting compounds: 6-(4-Amino-benzyl)-2,3,4,7-tetrahydro-indolo[2,3-c]quinolin-1-one (compound 38) and acetyl chloride
mp.: decomposes at 230-232°C
MS (MH⁺ found) = 384.3

### 43. 6-(4-Methoxy-benzyl)-3,3-dimethyl-2,3,4,7-tetrahydro-1 H-indolo[2,3-c]quinolin-1-ol

200 mg 6-(4-methoxy-benzyl)-3,3-dimethyl-2,3,4,7-tetrahydro-indolo[2,3-c]quinolin-1-one (compound 1) is dissolved in 10 ml methanol and treated with 50 mg NaBH₄. The reaction mixture is stirred for 2.5 h and treated again with 10 mg NaBH₄. The mixture is stirred for additional 2 h and evaporated. The residue is dissolved in 30 ml dichloromethane and 20 ml saturated aqueous NaHCO₃ solution. The organic layer is separated and the aqueous layer is extracted again with 30 ml dichloromethane. The combined organic layers are dried, evaporated and purified by flash chromatography to give 172 mg of the title compound.
¹H-NMR (200 MHz, CDCl₃): δ= 1.07 (s,3H), 1.22 (s,3H), 1.86-1.99 (m,2H), 2.23 (dd,J=13.7Hz,J=6.3Hz,1H), 2.85 (d,J=16.5Hz,1H), 3.05 (d,J=16.5Hz,1H), 3.77 (s,3H), 4.45 (s,2H), 5.56-5.59 (m,1H), 6.82-6.86 (m,2H), 7.20-7.34 (m,4H), 7.42-7.50 (m,1H), 7.74 (br,1H), 8.46 (d, J=8.0Hz, 1H)
mp.: 146°C

The following compounds are obtained by using the procedure of Example 43 analogously.

### 44. 6-(4-Methoxy-benzyl)-2,3,4,7-tetrahydro-1 H-indolo[2,3-c]quinolin-1-ol

Starting compound: 6-(4-Methoxy-benzyl)-2,3,4,7-tetrahydro-indolo[2,3-c]quinolin-1-one (compound 2)
¹H-NMR (200 MHz, CDCl₃): δ = 1.97-2.29 (m,5H), 2.94-3.10 (m,1H), 3.16-3.26 (m,1H), 3.77 (s,3H), 4.77 (s,2H), 5.56-5.65 (m,1H), 6.82-6.87 (m,2H), 7.21-7.36 (m,4H), 7.44-7.52 (m,1H), 7.74 (br,1H), 8.37 (d,J=8.1Hz,1H)
mp.: 204°C

### 45. 6-Benzyl-2,3,4,7-tetrahydro-1H-indolo[2,3-c]quinolin-1-ol

Starting compound: 6-Benzyl-2,3,4,7-tetrahydro-indolo[2,3-c]quinolin-1-one (compound 3)
¹H-NMR (200 MHz, D₆-DMSO): δ = 1.81-1.93 (m,2H), 2.03-2.14 (m,2H), 2.73-2.93 (m,2H), 4.40 (s,2H), 5.14 (d,J=6.5Hz,1H), 5.31-5.35 (m,1H), 7.09-7.27 (m,4H9, 7.34-7.38 (m,2H), 7.46-7.59 (m,2H), 8.35 (d,J=8.0Hz, 1H), 11.48 (br,1H)
mp.:211°C

### 46. 6-Benzyl-3,3-dimethyl-2,3,4,7-tetrahydro-1H-indolo[2,3-c]quinolin-1-ol

Starting compound: 6-Benzyl-3,3-dimethyl-2,3,4,7-tetrahydro-indolo[2,3-c]quinolin-1-one (compound B1)
¹H-NMR (200 MHz, CDCl₃): δ = 1.07 (s,3H), 1.22 (s,3H), 1.86-1.99 (m,2H), 2.23 (dd,J=13.7Hz,J=6.3Hz,1H), 2.85 (d,J=16.5Hz,1H), 3.05 (d,J=16.5Hz,1H), 4.41 (s,2H), 5.56-5.59 (m,1H), 7.21-7.33 (m,7H), 7.42-7.50 (m,1H), 7.74 (br,1H), 8.46 (d,J=8.0Hz,1H)

### 47. 6-(4-Hydroxy-benzyl)-3,3-dimethyl-2,3,4,7-tetrahydro-1H-indolo[2,3-c]quinolin-1-ol

Starting compound: 6-(4-Hydroxy-benzyl)-3,3-dimethyl-2,3,4,7-tetrahydro-indolo[2,3-c]quinolin-1-one (compound 35)
¹H-NMR (400 MHz, d₆-DMSO): δ = 0.93 (s, 3H), 1.12 (s,3H), 1.73-1.78 (m,1H), 2.01-2.06 (m,1H), 2.65(d,J=16Hz,1H), 2.84 (d,J=16Hz,1H),4.22 (d,J=14Hz,1H), 4.32 (d,J=14Hz,1H), 5.04 (br,1H), 5.27-5.31 (m,1H), 6.62 (d,J=8.4Hz,2H), 7.13-7.18 (m,3H), 7.46-7.49 (m,1H), 8.45 (d,J=8Hz,1H), 9.11 (s,1H), 11.47 (br,1H)
mp.: 212°C

### 48. 6-(4-Methoxy-benzyl)-3,3-dimethyl-5-oxy-2,3,4,7-tetrahydro-1H-indolo[2,3-c]quinolin-1-ol

Starting compound: 6-(4-Methoxy-benzyl)-3,3-dimethyl-5-oxy-2,3,4,7-tetrahydro-indolo[2,3-c]quinolin-1-one (compound 74)
¹H-NMR (400 MHz, CDCl₃): δ = 0.92 (s,3H), 1.24 (s,3H), 1.85-1.90 (m,1H), 2.18-2.23 (m,1H), 2.84 (d,J=18Hz,1H), 2.97 (d,J=18Hz,1H), 2.88 (d,J=16.7Hz,1H), , 3.72 (s,3H), 4.18 (br,1H), 4.26 (d,J=16.7Hz,1H), 5.26-5.31 (m,1H), 6.69 (d,J=8.5Hz,2H), 6.78 (d,J=8.5Hz,2H), 7.29-7.35 (m,2H), 7.47-7.51 (m,2H), 8.59 (d, J=8.1Hz, 1H)
mp.: 185°C

### 49. 3,3-Dimethyl-6-(4-trifluoromethoxy-benzyl)-2,3,4,7-tetrahydro-1 H-indolo[2,3-c]quinolin-1-ol

Starting compound: 3,3-Dimethyl-6-(4-trifluoromethoxy-benzyl)-2,3,4,7-tetrahydro-indolo[2,3-c]quinolin-1-one (compound 11)
¹H-NMR (200 MHz, DMSO-d6): δ= 0.93 (s,3H), 1.12 (s,3H), 1.70-1.85 (m,1H), 2.00-2.15 (m,1H), 2.65 (d,J=16.9Hz,1H), 2.85 (d,J=16.9Hz,1H), 4.37 (d,J=14.3Hz,1H), 4.48 (d,J=14.3Hz,1H), 5.08 (d,J=6.8Hz,1H), 5.31 (m,1H), 7.10-7.30 (m,3H), 7.40-7.60 (m,4H), 8.46 (d, J=8.0Hz, 1H), 11.58 (s,1H)
mp.: 117-119°C
MS (MH⁺ found) = 441.4

### 50. 6-(4-Amino-benzyl)-3,3-dimethyl-2,3,4,7-tetrahydro-1H-indolo[2,3-c]quinolin-1-ol

Starting compound: 6-(4-Amino-benzyl)-3,3-dimethyl-2,3,4,7-tetrahydro-indolo[2,3-c]quinolin-1-one (compound 36)
mp.: 206-208°C
MS (MH⁺ found) = 372.3

### 51. 6-(4-Ethoxy-benzyl)-3,3-dimethyl-2,3,4,7-tetrahydro-1H-indolo[2,3-c]quinolin-1-ol

Starting compound: 6-(4-Ethoxy-benzyl)-3,3-dimethyl-2,3,4,7-tetrahydro-indolo[2,3-c]quinolin-1-one (compound 12)
mp.: 182-184°C
MS (MH⁺ found) = 401.4

### 52. 3,3-Dimethyl-6-(3-nitro-benzyl)-2,3,4,7-tetrahydro-1H-indolo[2,3-c]quinolin-1-ol

Starting compound: 3,3-Dimethyl-6-(3-nitro-benzyl)-2,3,4,7-tetrahydro-indolo[2,3-c]quinolin-1-one (compound 13)
mp.: 228-230°C
MS (MH⁺ found) = 402.3

### 53. 6-(3-Amino-benzyl)-3,3-dimethyl-2,3,4,7-tetrahydro-1H-indolo[2,3-c]quinolin-1-ol

Starting compound: 6-(3-Amino-benzyl)-3,3-dimethyl-2,3,4,7-tetrahydro-indolo[2,3-c]quinolin-1-one (compound 37)
mp.: 225-227°C
MS (MH⁺ found) = 372.4

### 54. 6-(4-Isopropyl-benzyl)-3,3-dimethyl-2,3,4,7-tetrahydro-1H-indolo[2,3-c]quinolin-1-ol

Starting compound: 6-(4-Isopropyl-benzyl)-3,3-dimethyl-2,3,4,7-tetrahydro-indolo[2,3-c]quinolin-1-one (compound 14)
mp.: 112-114°C
MS (MH⁺ found) = 399.4

### 55. 6-Benzo[1,3]dioxol-5-ylmethyl-3,3-dimethyl-2,3,4,7-tetrahydro-1H-indolo[2,3-c]quinolin-1-ol

Starting compound: 6-Benzo[1,3]dioxol-5-ylmethyl-3,3-dimethyl-2,3,4,7-tetrahydro-indolo[2,3-c]quinolin-1-one, HCl salt (compound 7)
mp.: 114-119°C
MS (MH⁺ found) = 401.4

### 56. N-[4-(1-Hydroxy-3,3-dimethyl-2,3,4,7-tetrahydro-1H-indolo[2,3-c]quinolin-6-ylmethyl)-phenyl]-propionamide

Starting compound: N-[4-(3,3-Dimethyl-1-oxo-2,3,4,7-tetrahydro-1H-indolo[2,3-c]quinolin-6-ylmethyl)-phenyl]-propionamide (compound 41)
mp.: 165-168°C
MS (MH⁺ found) = 428.4

### 57. 6-Benzo[1,3]dioxol-5-ylmethyl-2,3,4,7-tetrahydro-1H-indolo[2,3-c]quinolin-1-ol

Starting compound: 6-Benzo[1,3]dioxol-5-ylmethyl-2,3,4,7-tetrahydro-indolo[2,3-c]quinolin-1-one (compound 18)
mp.: 120-123°C
MS (MH⁺ found) = 373.3

### 58. 6-(3-Bromo-benzyl)-3,3-dimethyl-2,3,4,7-tetrahydro-1 H-indolo[2,3-c]quinolin-1-ol

Starting compound: 6-(3-Bromo-benzyl)-3,3-dimethyl-2,3,4,7-tetrahydro-indolo[2,3-c]quinolin-1-one (compound 21)
mp.: 118-125°C
MS (MH⁺ found) = 435.3 and 437.3

### 59. N-[4-(1-Hydroxy-3,3-dimethyl-2,3,4,7-tetrahydro-1H-indolo[2,3-c]quinolin-6-ylmethyl)-phenyl]-acetamide

Starting compound: N-[4-(3,3-Dimethyl-1-oxo-2,3,4,7-tetrahydro-1H-indolo[2,3-c]quinolin-6-ylmethyl)-phenyl]-acetamide (compound 40)
mp.: 195-197°C
MS (MH⁺ found) = 414.4

### 60. 6-(4-Fluoro-benzyl)-3,3-dimethyl-2,3,4,7-tetrahydro-1H-indolo[2,3-c]quinolin-1-ol

Starting compound: 6-(4-Fluoro-benzyl)-3,3-dimethyl-2,3,4,7-tetrahydro-indolo[2,3-c]quinolin-1-one (compound 28)
mp.: 187-189°C
MS (MH⁺ found) = 375.4

### 61. 6-(2-Fluoro-4-methoxy-benzyl)-3,3-dimethyl-2,3,4,7-tetrahydro-1 H-indolo[2,3-c]quinolin-1-ol

Starting compound: 6-(2-Fluoro-4-methoxy-benzyl)-3,3-dimethyl-2,3,4,7-tetrahydro-indolo[2,3-c]quinolin-1-one (compound 27)
MS (MH⁺ found) = 405.3

### 62. 6-(3,4-Difluoro-benzyl)-3,3-dimethyl-2,3,4,7-tetrahydro-1 H-indolo[2,3-c]quinolin-1-ol

Starting compound: 6-(3,4-Difluoro-benzyl)-3,3-dimethyl-2,3,4,7-tetrahydro-indolo[2,3-c]quinolin-1-one (compound 24)
mp.: 121-128°C
MS (MH⁺ found) = 393.4

### 63. 6-(3,5-Difluoro-benzyl)-3,3-dimethyl-2,3,4,7-tetrahydro-1 H-indolo[2,3-c]quinolin-1-ol

Starting compound: 6-(3,5-Difluoro-benzyl)-3,3-dimethyl-2,3,4,7-tetrahydro-indolo[2,3-c]quinolin-1-one (compound 22)
mp.: 117-123°C
MS (MH⁺ found) = 393.4

### 64. 6-(3-Fluoro-4-methoxy-benzyl)-3,3-dimethyl-2,3,4,7-tetrahydro-1H-indolo[2,3-c]quinolin-1-ol

Starting compound: 6-(3-Fluoro-4-methoxy-benzyl)-3,3-dimethyl-2,3,4,7-tetrahydro-indolo[2,3-c]quinolin-1-one (compound 23)
MS (MH⁺ found) = 405.4

### 65. N-[4-(1-Hydroxy-2,3,4,7-tetrahydro-1H-indolo[2,3-c]quinolin-6-ylmethyl)-phenyl]-acetamide

Starting compound: N-[4-(1-Oxo-2,3,4,7-tetrahydro-1H-indolo[2,3-c]quinolin-6-ylmethyl)-phenyl]-acetamide (compound 42)
mp.: 195-197°C
MS (MH⁺ found) = 386.3

### 66. [4-(1-Hydroxy-3,3-dimethyl-2,3,4,7-tetrahydro-1H-indolo[2,3-c]quinolin-6-ylmethyl)-phenyl]-urea

Starting compound: [4-(3,3-Dimethyl-1-oxo-2,3,4,7-tetrahydro-1H-indolo[2,3-c]quinolin-6-ylmethyl)-phenyl]-urea (compound 71)
mp.: 236-238°C
MS (MH⁺ found) = 415.3

### 67. 6-(3-Fluoro-4-methoxy-benzyl)-2,3,4,7-tetrahydro-1 H-indolo[2,3-c]quinolin-1-ol

Starting compound: 6-(3-Fluoro-4-methoxy-benzyl)-2,3,4,7-tetrahydro-indolo[2,3-c]quinolin-1-one (compound 29)
mp.: 118-120°C
MS (MH⁺ found) = 377.3

### 68. 6-(3-Amino-benzyl)-2,3,4,7-tetrahydro-1H-indolo[2,3-c]quinolin-1-ol

Starting compound: 6-(3-Amino-benzyl)-2,3,4,7-tetrahydro-indolo[2,3-c]quinolin-1-one (compound 39)
MS (MH⁺ found) = 344.3

### 69. 6-(3,5-Difluoro-4-methoxy-benzyl)-3,3-dimethyl-2,3,4,7-tetrahydro-1H-indolo[2,3-c]quinolin-1-ol

Starting compound: 6-(3,5-Difluoro-4-methoxy-benzyl)-3,3-dimethyl-2,3,4,7-tetrahydro-indolo[2,3-c]quinolin-1-one (compound 32)
mp.: 131-133°C
MS (MH⁺ found) = 423.4

### 70. 6-(2,3-Difluoro-4-methoxy-benzyl)-3,3-dimethyl-2,3,4,7-tetrahydro-1H-indolo[2,3-c]quinolin-1-ol

Starting compound: 6-(2,3-Difluoro-4-methoxy-benzyl)-3,3-dimethyl-2,3,4,7-tetrahydro-indolo[2,3-c]quinolin-1-one (compound 31)
mp.: 197-198°C
MS (MH⁺ found) = 423.4

### 71. [4-(3,3-Dimethyl-1-oxo-2,3,4,7-tetrahydro-1H-indolo[2,3-c]quinolin-6-ylmethyl)-phenyl]-urea

0.37 g 6-(4-amino-benzyl)-3,3-dimethyl-2,3,4,7-tetrahydro-indolo[2,3-c]quinolin-1-one (compound 36) is dissolved in 40 ml water and 1 ml concentrated hydrochloric acid. During 8 h, a solution of 0.81 g KNCO in 10 ml water is added using a syringe pump and the mixture is stirred for additional 17 h at RT. The mixture is poured in 20 ml 1 M Na₂CO₃ solution and extracted with THF / ethyl acetate (1/1 v/v). The organic layer is washed with brine, dried with MgSO₄ and concentrated in vacuo. The product is recrystallized from ethyl acetate to yield 0.30 g of the title compound. mp.: decomposes at 235°C
MS (MH⁺ found) = 413.3

The following compound is obtained using the procedure of Example 71 analogously:

### 72. [4-(1-Oxo-2,3,4,7-tetrahydro-1H-indolo[2,3-c]quinolin-6-ylmethyl)-phenyl]-urea

Starting compound: 6-(4-Amino-benzyl)-2,3,4,7-tetrahydro-indolo[2,3-c]quinolin-1-one (compound 38)
mp.: 232-234°C
MS (MH⁺ found) = 385.2

### 73. 6-(4-Methoxy-benzyl)-3,3-dimethyl-2,3,4,7-tetrahydro-1 H-indolo[2,3-c]quinoline

385 mg 6-(4-methoxy-benzyl)-3,3-dimethyl-2,3,4,7-tetrahydro-indolo[2,3-c]quinolin-1-one (compound 1) is solved in 1.35 ml triethylene glycol and treated with 160 µl 85% (w/v) aqueous hydrazine solution. The reaction mixture is heated to 130°C for 20 h. The mixture is diluted with dichloromethane and water and acidified with aqueous HCl solution. The organic layer is separated and the aqueous layer is extracted. The extracts are dried and concentrated in vacuo. The residue is purified by flash chromatography to give 221 mg of the title compound.
¹H-NMR (200 MHz, D₆-DMSO): δ = 1.02 (s,6H), 1.70 (t,J=6.5Hz,2H), 2.72 (s,2H), 3.26 (t,J=6.5Hz,2H), 3.67 (s,3H), 4.30 (s,2H), 6.78-6.83 (m,2H), 7.16-7.29 (m,3H), 7.46-7.60 (m,2H), 8.15 (d,J=7.9Hz,1H), 11.47 (br,1H)
mp.: 158°C

### 74. 6-(4-Methoxy-benzyl)-3,3-dimethyl-5-oxy-2,3,4,7-tetrahydro-indolo[2,3-c]quinolin-1-one

100 mg 6-(4-methoxy-benzyl)-3,3-dimethyl-2,3,4,7-tetrahydro-indolo[2,3-c]quinolin-1-one (compound 1) is suspended in diethylether and 54 mg m-chloro perbenzoic acid is added. The reaction mixture is stirred for 16 h. Then, additional 25 mg m-chloro perbenzoic acid is added and the reaction mixture is stirred for further 12 h. 10 ml water and 10 ml dichloromethane are added and the mixture is worked up extractively. After evaporation, the residue is crystallized from diisopropylether to give 82 mg of the title compound.
¹H-NMR (200 MHz, d₆-DMSO): δ = 1.1 (s,6H), 2.69 (s,2H), 3.12 (s,2H), 3.67 (s,3H), 4.58 (s,2H), 6.79-6.83 (m,2H), 7.21-7.35 (m,3H), 7.50-7.63 (m,2H), 9.07 (d, J=7.6Hz,1H), 12.1 (br, 1H)
mp.: 202°C

### 75. (S)-6-(3-Fluoro-4-methoxy-benzyl)-3,3-dimethyl-2,3,4,7-tetrahydro-1H-indolo[2,3-c]quinolin-1-ol

0.50 g 3-nitrophenylboronic acid, 0.45 g D-tartaric acid and 0.25 g calcium hydride are suspended in 10 ml THF and heated under reflux for 2 h. The suspension is cooled and filtered under nitrogen atmosphere. 0.41 g 6-(3-fluoro-4-methoxy-benzyl)-3,3-dimethyl-2,3,4,7-tetrahydro-indolo[2,3-c]quinolin-1-one (compound 23) is dissolved in the filtrate and 0.080 g NaBH₄ is added portionwise. After the last addition, stirring is continued for 30 min at RT. 5 ml Methanol is added slowly (hydrogen evolution) and the mixture is stirred for 30 min. Next, 5 ml 2M hydrochloric acid is added and stirring is continued for 1 h. The mixture is basified with 2M NaOH and extracted with 15 ml DCM and 25 ml water. The water layer is extracted twice with 15 ml DCM. The DCM layers are washed with 20 ml water, dried with MgSO₄ and concentrated in vacuo. The product is purified by chromatography (Silicagel, DCM / EA 9/1 v/v). Trituration with ether gave 0.27 g white crystals.
¹H-NMR (200 MHz, CDCl₃): δ= 1.00 (s,3H), 1.15 (s,3H), 1.80-2.00 (m,1H+OH), 2.15-2.30 (m,1H), 2.77 (d,J=16.5,1H), 2.97 (d,J=16.5,1H), 3.77 (s,3H), 4.34 (s,2H), 6.75-7.00 (m,3H), 7.26 (t,J=8.2Hz,1H), 7.35 (d,J=B.OHz,1H), 7.42 (t,J=7.OHz,1H), 7.81 (s,1H), 8.40 (d,J=8.1Hz,1H)
mp.: 164-166 °C
MS (MH⁺ found) = 405.4
ee > 98% [ChiralCel OD-RH column (150 x 4.6 mm, 5 micrometer). Eluent: acetonitrile/water (60/40 v/v) with 2 Vol% triethylamine. Flow 0.5 ml/min. Rf (S-isomer) = 10.1 min. Rf (R-isomer) = 9.0 min.]

The following compounds are obtained in accordance with the procedure of Example 75 (D-tartaric acid gives the S-isomer and L-tartaric acid gives the R-isomer):

### 76. (R)-6-(4-Methoxy-benzyl)-3,3-dimethyl-2,3,4,7-tetrahydro-1H-indolo[2,3-c]quinolin-1-ol

Starting compound: 6-(4-Methoxy-benzyl)-3,3-dimethyl-2,3,4,7-tetrahydro-indolo[2,3-c]quinolin-1-one (compound 1)
mp.: 118-120°C
MS (MH⁺ found) = 387.4

### 77. (S)-6-(4-Methoxy-benzyl)-3,3-dimethyl-2,3,4,7-tetrahydro-1H-indolo[2,3-c]quinolin-1-ol

Starting compound: 6-(4-Methoxy-benzyl)-3,3-dimethyl-2,3,4,7-tetrahydro-indolo[2,3-c]quinolin-1-one (compound 1)
mp.: 118-120°C
MS (MH⁺ found) = 387.4

### 78. (R)-6-(3-Fluoro-4-methoxy-benzyl)-3,3-dimethyl-2,3,4,7-tetrahydro-1H-indolo[2,3-c]quinolin-1-ol

Starting compound: 6-(3-Fluoro-4-methoxy-benzyl)-3,3-dimethyl-2,3,4,7-tetrahydro-indolo[2,3-c]quinolin-1-one (compound 23)
mp.: 164-166°C
MS (MH⁺ found) = 405.4

### 79. (R)-6-(3-Fluoro-4-methoxy-benzyl)-2,3,4,7-tetrahydro-1H-indolo[2,3-c]quinolin-1-ol

Starting compound: 6-(3-Fluoro-4-methoxy-benzyl)-2,3,4,7-tetrahydro-indolo[2,3-c]quinolin-1-one (compound 29)
mp.: 124-127°C
MS (MH⁺ found) = 377.4

### 80. (R)-6-(3,5-Difluoro-benzyl)-3,3-dimethyl-2,3,4,7-tetrahydro-1 H-indolo[2,3-c]quinolin-1-ol

Starting compound: 6-(3,5-Difluoro-benzyl)-3,3-dimethyl-2,3,4,7-tetrahydro-indolo[2,3-c]quinolin-1-one (compound 22)
mp.: 129-131°C
MS (MH⁺ found) = 393.4

### 81. (S)-6-(3-Fluoro-4-methoxy-benzyl)-2,3,4,7-tetrahydro-1H-indolo[2,3-c]quinolin-1-ol

Starting compound: 6-(3-Fluoro-4-methoxy-benzyl)-2,3,4,7-tetrahydro-indolo[2,3-c]quinolin-1-one (compound 29)
mp.: 125-127°C
MS (MH⁺ found) = 377.4

### 82. (S)-6-(3,5-Difluoro-benzyl)-3,3-dimethyl-2,3,4,7-tetrahydro-1H-indolo[2,3-c]quinolin-1-ol

Starting compound: 6-(3,5-Difluoro-benzyl)-3,3-dimethyl-2,3,4,7-tetrahydro-indolo[2,3-c]quinolin-1-one (compound 22)
mp.: 130-131°C
MS (MH⁺ found) = 393.4

### 83. (R)-6-(3-Fluoro-4-hydroxy-benzyl)-3,3-dimethyl-2,3,4,7-tetrahydro-1H-indolo[2,3-c]quinolin-1-ol

Starting compound: 6-(3-Fluoro-4-hydroxy-benzyl)-3,3-dimethyl-2,3,4,7-tetrahydro-indolo[2,3-c]quinolin-1-one (compound 34)
mp.: 232-234°C.
MS (MH⁺ found) = 391.4

### 84. (S)-6-(3-Fluoro-4-hydroxy-benzyl)-3,3-dimethyl-2,3,4,7-tetrahydro-1H-indolo[2,3-c]quinolin-1-ol

Starting compound: 6-(3-Fluoro-4-hydroxy-benzyl)-3,3-dimethyl-2,3,4,7-tetrahydro-indolo[2,3-c]quinolin-1-one (compound 34)
mp.: 233-234°C.
MS (MH⁺ found) = 391.4

### 85. (S)-6-(3,5-Difluoro-4-methoxy-benzyl)-3,3-dimethyl-2,3,4,7-tetrahydro-1 H-indolo[2,3-c]quinolin-1-ol

Starting compound: 6-(3,5-Difluoro-4-methoxy-benzyl)-3,3-dimethyl-2,3,4,7-tetrahydro-indolo[2,3-c]quinolin-1-one (compound 32)
mp.: 161-162°C.
MS (MH⁺ found) = 423.4

### 86. (R)-6-(3,5-Difluoro-4-methoxy-benzyl)-3,3-dimethyl-2,3,4,7-tetrahydro-1 H-indolo[2,3-c]quinolin-1-ol

Starting compound: 6-(3,5-Difluoro-4-methoxy-benzyl)-3,3-dimethyl-2,3,4,7-tetrahydro-indolo[2,3-c]quinolin-1-one (compound 32)
mp.: 161-162°C.
MS (MH⁺ found) = 423.4

### 87. (S)-6-(2,3-Difluoro-4-methoxy-benzyl)-3,3-dimethyl-2,3,4,7-tetrahydro-1 H-indolo[2,3-c]quinolin-1-ol

Starting compound: 6-(2,3-Difluoro-4-methoxy-benzyl)-3,3-dimethyl-2,3,4,7-tetrahydro-indolo[2,3-c]quinolin-1-one (compound 31)

### 88. (S)-6-(2,3-Dihydro-benzofuran-5-ylmethyl)-3,3-dimethyl-2,3,4,7-tetrahydro-1 H-indolo[2,3-c]quinolin-1-ol

Starting compound: 6-(2,3-Dihydro-benzofuran-5-ylmethyl)-3,3-dimethyl-2,3,4,7-tetrahydro-indolo[2,3-c]quinolin-1-one (compound 33)

### Starting Compounds

### A1. 3-Hyd roxy-2-(1H-indol-3-yl)-5,5-dimethyl-cyclohex-2-enone

Step 1: 10.5 g 1-acetyl-1,2-dihydro-indol-3-one is suspended in 50 ml of acetic acid and a suspension of 8.4 g 5,5-dimethyl-cyclohexane-1,3-dione in 50 ml acetic acid is added. The reaction mixture is stirred for 20 min and 8.3 ml triethylamine is added slowly. The mixture is refluxed for 24 h, evaporated to dryness, treated with 30 ml of 2N methanolic HCl solution and evaporated to dryness again. The residue is purified by flash chromatography and crystallized from diethyl ether to give 11.5 g of 2-(1-acetyl-1H-indol-1-yl)-3-hydroxy-5,5-dimethyl-cyclohex-2-enone.
¹H-NMR (200 MHz, D₆-DMSO): δ = 1.13 (s,6H), 2.42 (br, 4H), 2.61 (s, 3H), 7.17-7.19 (m,2H), 7.23-7.31 (m, 1H), 8.31 (d, J=7.8Hz,1 H)
mp.: 230°C
Step 2: 5.95 g 2-(1-acetyl-1H-indol-1-yl)-3-hydroxy-5,5-dimethyl-cyclohex-2-enone is dissolved in 65 ml of aqueous 1 N NaOH solution and stirred for 2 h. The reaction mixture is diluted with dichloromethane and acidified with aqueous HCl solution (pH 5). The organic layer is separated and the aqueous layer is extracted again with dichloromethane. The combined organic layers are dried and concentrated to give 5.09 g of the title compound.
¹H-NMR (200 MHz, CDCl₃): δ = 1.22 (s,6H), 2.50 (s, 4H), 7.00 (d,J=2.5Hz,1H), 7.06-7.21 (m,2H), 7.25-7.32 (m,2H9, 8.73 (br,1 H)
mp.: 178°C

The following compounds are obtained by using the procedure of Example A1 analogously:

### A2. 3-Hydroxy-2-(1H-indol-3-yl)-5-methyl-cyclohex-2-enone

Starting compound: 5-Methyl-cyclohexane-1,3-dione
mp.: 127-129°C

### A3. 3-Hydroxy-2-(1H-indol-3-yl)-cyclohex-2-enone

Starting compound: Cyclohexane-1,3-dione
¹H-NMR (200 MHz, CDCl3): δ= 2.06-2.20 (m,2H), 2.59-2.65 (m,4H), 7.07-7.43 (m,5H), 8.61 (br,1 H)
mp.: 151-153°C

### A4. 2-(1 H-indol-3-yl)-cyclohexanone

3.5 g indole is added to a mixture of 25 ml glacial acetic acid and 6.5 ml 2N H₃PO₄ at 135°C followed by 5.0 g 2-hydroxycyclohexanone dimer and refluxed for 30 min. The reaction mixture is cooled in ice and poured into 75 ml concentrated ammonia cooled in an ice-bath. The suspension is extracted with 50 ml ethyl acetate three times. The ethyl acetate layers are washed with 50 ml water, dried with MgSO₄ and concentrated in vacuo. The product is purified by chromatography (Silicagel, DCM / EA 19/1 v/v) and crystallized from ethanol to give 3.2 g of the title compound.
¹H-NMR (200 MHz, CDCl₃): δ = 1.70-2.60 (m, 8H), 3.90 (dd, J=5.2 and 10.8Hz,1H), 7.00-7.25 (m,3H), 7.32 (d, J=8.0Hz,1H), 7.43 (d, J=7.6Hz,1H), 8.07 (brs,NH)
mp.: 122-124°C

### A5. 2-(1 H-Indol-3-yl)-5-methyl-cyclohexanone

7.03 g indole and 3.68 ml 3-methyl cyclohexanone are dissolved in THF and cooled to -75°C. 90 ml of a 1 M solution of lithium hexamethyldisilazide (LHMDS) in THF is added slowly and the solution is stirred for 30 min. Copper 2-ethyl hexanoate is added in one portion and the reaction mixture is stirred for 16 h at -75°C. The mixture is warmed to room temperature, poured into 300 ml 1 N HCl solution and worked up extractively. The combined organic layers are evaporated and the residue is purified by flash chromatography to give 750 mg of the title compound.
¹H-NMR (400 MHz, d₆-DMSO): δ = 0.97 (d,J=6.Hz,1.5H),1.05 (d,J=6.5Hz,1.5H), 1.57-1.62 (m,1H), 1.89-1.93 (m,1H), 2.08-2.36 (m,4H), 3.83-3.91 (m,1H), 6.89-6.95 (m,1H), 7.01-7.07 (m,1H), 7.12 (d,J=2.1 Hz,0.5H), 7.26 (d,J=2.1 Hz,0.5H), 7.31-7.37 (m,2H), 10.83 (br,0.5H), 10.97 (br,0.5H)

### B1. 6-Benzyl-3,3-dimethyl-2,3,4,7-tetrahydro-indolo[2,3-c]quinolin-1-one

The compound is prepared analogously to Example 1 from 3-hydroxy-2-(1H-indol-3-yl)-5,5-dimethyl-cyclohex-2-enone (compound A1) and phenyl acetic acid anhydride.
¹H-NMR (200 MHz, CDCl₃): δ= 1.19 (s,6H), 2.72 (s,2H), 3.26 (s,2H), 4.57 (s,2H), 7.24-7.34 (m,7H), 7.90 (br,1H), 9.33 (d,J=8.3Hz,1H)
mp.: 193°C

### Commercial utility

The compounds, salts thereof, N-oxides of the compounds and the salts thereof, and the stereoisomers of the compounds, the salts, the N-oxides of the compounds and the N-oxides of the salts thereof according to the invention are hereinafter referred to as the compounds of the invention. In particular, the compounds of the invention are pharmaceutically acceptable.

The compounds of the invention have valuable pharmaceutical properties which make them commercially utilizable. In particular, as type 5 phosphodiesterase (PDE5) inhibitors, they are able to influence the physiological and pathophysiological function of various cells, e.g., but not limited to, smooth muscle cells, fibroblasts, myofibroblasts and platelets, which are involved in a great variety of physiological and pathophysiological mechanisms. In particular, the PDE5 inhibiting compounds of the invention can effect relaxation of the vasculature, thus increasing blood flow, improve the spatial balance between blood perfusion and ventilation within the lung ("re-matching" effect) thereby reducing the amount of so-called low V/Q-areas [areas within the lung with high perfusion (Q) but no or reduced ventilation (V)] and high V/Q-areas (areas within the lung with low perfusion but high ventilation), induce neurogenesis, inhibit platelet function, such as aggregation, adhesion and mediator release and, thus, have an anti-inflammatory effect. The compounds of the invention are distinguished by valuable and desirable properties, such as, for example, high efficacy, high selectivity, low toxicity, superior bioavailability in general (e.g. good enteral absorption), superior therapeutic window, superior pharmacokinetics (e.g. half-life), absence of significant side effects, and further beneficial effects related with their therapeutic and pharmaceutical suitability.

Accordingly, the invention further relates to the compounds of the invention for use in the treatment or prophylaxis of diseases, especially diseases alleviated by inhibition of the type 5 phosphodiesterase. In particular, the invention relates to the compounds of the invention for use in the treatment or prophylaxis of the following diseases:
male and female sexual dysfunction, such as, but not limited to, male erectile dysfunction, premature ejaculation, Peyronie's disease;
acute and chronic airway diseases, such as, but not limited to, COPD (chronic obstructive pulmonary disease), bronchitis, emphysema, pulmonary vascular remodeling, pulmonary hypertension, lung fibrosis, asthma, cystic fibrosis, bronchiectasis, bronchiolitis obliterans, connective tissue diseases, sarcoidosis, kyphoscoliosis, pneumoconiosis, amyotrophic lateral sclerosis, thoracoplasty, extrinsic allergic alveolitis;
inflammatory diseases, such as, but not limited to, vasculature inflammation, acute respiratory distress syndrome, mesangial glomerulonephritis, chronic inflammatory bowel disease, disseminated intravascular inflammation, allergic vasculitis, dermatoses (e.g., but not limited to, psoriasis, toxic and allergic contact eczema, atopic eczema, seborrhoeic eczema, Lichen simplex, sunburn, pruritus in the anogenital area, alopecia areata, hypertrophic scars, discoid lupus erythematosus, follicular and widespread pyodermias, endogenous and exogenous acne, acne rosacea), disorders of the arthritis type (e.g., but not limited to, rheumatoid arthritis, rheumatoid spondylitis, osteoarthritis), disorders of the immune system [e.g., but not limited to, AIDS (acquired immunodeficiency syndrome), multiple sclerosis], graft versus host reaction, allograft rejections, shock [e.g., but not limited to, septic shock, endotoxin shock, gram-negative sepsis shock, toxic shock syndrome and ARDS (adult respiratory distress syndrome)], gastrointestinal inflammations (e.g., but not limited to, Crohn's disease and ulcerative colitis); disorders which are based on allergic and/or chronic, immunological false reactions (e.g., but not limited to, allergic rhinitis, allergic sinusitis, chronic rhinitis, chronic sinusitis, allergic conjunctivitis, nasal polyps);
pain, such as, but not limited to, inflammatory pain;
right-heart failure, right heart hypertrophy (cor pulmonale), hypertension, hypercholesterolemia, hypertriglyceridemia;
ischaemic diseases, such as, but not limited to, diabetes mellitus, stroke, coronary artery disease, angina (including, but not limited to, vasospastic angina), myocardial infarction, peripheral artery disease, cerebrovascular obstruction, sleep apnea, macular ischaemia, arterial and venous occlusion, congestive heart failure;
diabetic gastroparesis and diseases with symptoms of gastroparesis;
diseases or conditions in which it is desirable to suppress platelet function, for example, but not limited to, after stent implantations (e.g., but not limited to, coronary stenting), after bypass operations, in pulmonary hypertension, thrombotic diseases, post-angioplasty stenosis, coronary artery disease, infarction (e.g., but not limited to, myocardial infarction), instable angina pectoris, stroke, and arterial and venous occlusion diseases (e.g., but not limited to, claudicatio intermittens); diseases or conditions with an impairment or dysfunction of cerebral vascular reactivity and/or neurovascular coupling, such as, but not limited to, arteriosclerotic dementia, multi-infarct dementia, cerebral senility;
diseases which are based on neuronal damage or degradation, such as but not limited to, stroke, spinal cord injury, brain injury, morbus parkinson, amyotrophic lateral sclerosis, morbus alzheimer, amyloidosis, prion diseases and neuropathy;
peripheral arterial diseases, chronic renal failure, chronic heart failure, sepsis, senile dementia (Alzheimer's disease), Creutzfeld-Jacob disease, septic encephalopathy, arteriosclerotic encephalopathy, diabetes associated encephalopathy, toxic encephalopathy, vascular and neuronal dementia, Huntington's disease, Parkinson's disease, multiple sclerosis and preeclampsia;
portal hypertension, liver cirrhosis, toxic liver damage (e.g., but not limited to, alcohol-induced liver damage), hepatitis, thrombosis of the portal vein, Budd-Chiari syndrome, malformation of liver veins, compression of liver veins (e.g., but without limitation, due to tumors), arteriovenous fistula,
diseases associated with an enlarged spleen, schistosomiasis (bilharziosis), sarcoidosis and other granulomatous diseases, primary biliary cirrhosis, myeloproliferative disorders (e.g., but not limited to, chronic myeloid leukemia, osteomyelofibrosis), lymphatic systemic diseases, collagenosis (e.g., but not limited to, systemic lupus erythematodes, sclerodermia), morbus Osler (congenital arteriovenous malformations, inter alia in the liver), nodular regenerative hyperplasia, tricuspid insufficiency, pericarditis constrictiva, veno-occlusive disease (VOD), non-alcoholic steatohepatitis (NASH), liver fibrosis;
benign prostatic hyperplasia;
insufficient uteroplacental blood flow in pregnancies with fetal growth restriction;
insufficient brain skills, such as but not limited to, verbal attainment, attention, concentration, deductive thinking, central auditory processing, cognition, learning, vigilance, apprehension and reagibility.

In this respect, the term "pulmonary hypertension" in particular embraces
- pulmonary arterial hypertension including primary pulmonary hypertension (e.g. sporadic or familial) and pulmonary arterial hypertension related, for example, but without limitation, to collagen vascular disease, congenital systemic-to-pulmonary shunts, portal hypertension, human immunodeficiency virus infection, drugs or toxins (e.g., but not limited to, anorexigens), persistent pulmonary hypertension of the newborn;
- pulmonary venous hypertension due to, for example, but without limitation, left-sided atrial or ventricular heart disease, left-sided valvular heart disease, extrinsic compression of central pulmonary veins (e.g. fibrosing mediastinitis, adenopathy in relation to tumors), pulmonary veno-occlusive disease;
- pulmonary hypertension associated with disorders of the respiratory system or hypoxemia including, for example, but without limitation, chronic obstructive pulmonary disease (COPD), interstitial lung disease, sleep-disordered breathing, alveolar hypoventilation disorders, chronic exposure to high altitude, neonatal lung disease, alveolar-capillary dysplasia;
- pulmonary hypertension caused by chronic thrombotic or embolic diseases including thromboembolic obstruction of proximal pulmonary arteries and obstruction of distal pulmonary arteries, such as pulmonary embolism (due to thrombus, tumor, ova, parasites, or foreign material), in situ thrombosis and sickle-cell disease;
- pulmonary hypertension caused by disorders directly affecting the pulmonary vasculature including inflammatory disorders (e.g., but not limited to, schistosomiasis, sarcoidosis) and pulmonary capillary hemangiomatosis.

Preferably, the invention further relates to the compounds of the invention for use in the treatment or prophylaxis of the following diseases: acute and chronic airway diseases, such as pulmonary hypertension, lung fibrosis, asthma, bronchitis, emphysema and chronic obstructive pulmonary disease; portal hypertension, liver cirrhosis, toxic liver damage, hepatitis, non-alcoholic steatohepatitis and liver fibrosis.

The invention also relates to the use of a compound of the invention in the manufacture of a pharmaceutical composition inhibiting the type 5 phosphodiesterase, in particular a pharmaceutical composition for the treatment or prophylaxis of diseases alleviated by inhibition of the type 5 phosphodiesterase, preferably, a pharmaceutical composition for the treatment or prophylaxis of the diseases exemplified above.

In particular, the invention relates to the use of a compound of the invention in the manufacture of a pharmaceutical composition for the treatment or prophylaxis of an acute or chronic airway disease, such as, but not limited to, pulmonary hypertension, lung fibrosis, asthma, bronchitis, emphysema and chronic obstructive pulmonary disease.

Furthermore, the invention relates to the use of a compound of the invention in the manufacture of a pharmaceutical composition for the treatment or prophylaxis of portal hypertension, liver cirrhosis, toxic liver damage, hepatitis, non-alcoholic steatohepatitis or liver fibrosis.

The invention further relates to a method of treating or preventing a disease comprising administering to a patient in need thereof a therapeutically effective amount of at least one of the compounds of the invention.

In particular, the invention relates to a method of treating or preventing one of the above mentioned diseases comprising administering to a patient in need thereof a therapeutically effective amount of at least one of the compounds of the invention.

Especially, the invention relates to a method of treating or preventing a disease which is alleviated by inhibition of the type 5 phosphodiesterase comprising administering to a patient in need thereof a therapeutically effective amount of at least one of the compounds of the invention.

Preferably, the invention relates to a method of treating or preventing an acute or chronic airway disease, for example, but not limited to, pulmonary hypertension, lung fibrosis, asthma, bronchitis, emphysema and chronic obstructive pulmonary disease, comprising administering to a patient in need thereof a therapeutically effective amount of at least one of the compounds of the invention.

Furthermore, the invention preferably relates to a method of treating or preventing portal hypertension, liver cirrhosis, toxic liver damage, hepatitis, non-alcoholic steatohepatitis or liver fibrosis comprising administering to a patient in need thereof a therapeutically effective amount of at least one of the compounds of the invention.

In the above methods, the patient is preferably a mammal, more preferably a human. Furthermore, in the above methods, at least one of the compounds of the invention can be used. Preferably, one or two of the compounds of the invention are used, more preferably, one of the compounds of the invention is used.

In a particularly preferred embodiment of the invention, the above methods of treating or preventing one of the above mentioned diseases comprise administering to a patient in need thereof a therapeutically effective amount of one compound of the examples according to the present invention.

The invention furthermore relates to a pharmaceutical composition which comprises at least one of the compounds of the invention together with at least one pharmaceutically acceptable auxiliary.

Preferably, the pharmaceutical composition comprises one or two of the compounds of the invention. More preferably, the pharmaceutical composition comprises one of the compounds of the invention.

In a particularly preferred embodiment of the invention, the pharmaceutical composition comprises a compound of the examples according to the present invention together with at least one pharmaceutically acceptable auxiliary.

The invention additionally relates to a pharmaceutical composition comprising at least one of the compounds of the invention, at least one pharmaceutically acceptable auxiliary and at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics.

In this respect, the therapeutic agent includes the corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics in form of the free compounds, the pharmaceutically acceptable salts thereof, the pharmaceutically acceptable derivatives thereof (e.g., but not limited to, ester derivatives), the solvates thereof and the stereoisomers of the compounds, salts, derivatives and solvates.

In a preferred embodiment, the pharmaceutical composition comprises a compound of the invention in combination with a corticosteroid. In a particularly preferred embodiment, the pharmaceutical composition comprises:
a compound of the invention and budesonide,
a compound of the invention and fluticasone,
a compound of the invention and beclometasone,
a compound of the invention and triamcinolone acetonide, or
a compound of the invention and ciclesonide.

In a further preferred embodiment, the pharmaceutical composition comprises a compound of the invention in combination with an anticholinergic. In a particularly preferred embodiment, the pharmaceutical composition comprises:
a compound of the invention and indacaterol,
a compound of the invention and tiotropium bromide, or
a compound of the invention and ipratropium bromide.

In a further preferred embodiment, the pharmaceutical composition comprises a compound of the invention in combination with a beta-mimetic. In a particularly preferred embodiment, the pharmaceutical composition comprises:
a compound of the invention and formoterol, or
a compound of the invention and salmeterol.

In a further preferred embodiment, the pharmaceutical composition comprises a compound of the invention in combination with a lung surfactant. In a particularly preferred embodiment, the pharmaceutical composition comprises:
a compound of the invention and lusupultide,
a compound of the invention and poractant alfa,
a compound of the invention and sinapultide,
a compound of the invention and beractant,
a compound of the invention and bovactant,
a compound of the invention and colfosceril palmitate,
a compound of the invention and surfactant-TA, or
a compound of the invention and calfactant.

In a further preferred embodiment, the pharmaceutical composition comprises a compound of the invention in combination with an endothelin antagonist. In a particularly preferred embodiment, the pharmaceutical composition comprises:
a compound of the invention and bosentan,
a compound of the invention and ambrisentan, or
a compound of the invention and sitaxsentan.

In a further preferred embodiment, the pharmaceutical composition comprises a compound of the invention in combination with a prostacyclin. In a particularly preferred embodiment, the pharmaceutical composition comprises:
a compound of the invention and iloprost,
a compound of the invention and epoprostenol,
a compound of the invention and triprostinil.

In a further preferred embodiment, the pharmaceutical composition comprises a compound of the invention in combination with a calcium channel blocker. In a particularly preferred embodiment, the pharmaceutical composition comprises:
a compound of the invention and amlodipine,
a compound of the invention and nifedipine,
a compound of the invention and diltiazem,
a compound of the invention and verapamil, or
a compound of the invention and felodipine.

In a further preferred embodiment, the pharmaceutical composition comprises a compound of the invention in combination with a beta-blocker. In a particularly preferred embodiment, the pharmaceutical composition comprises:
a compound of the invention and bisoprolol,
a compound of the invention and nebivolol,
a compound of the invention and metoprolol,
a compound of the invention and carvedilol,
a compound of the invention and atenolol, or
a compound of the invention and nadolol.

In a further preferred embodiment, the pharmaceutical composition comprises a compound of the invention in combination with a type 4 phosphodiesterase inhibitor. In a particularly preferred embodiment, the pharmaceutical composition comprises:
a compound of the invention and roflumilast,
a compound of the invention and roflumilast N-oxide,
a compound of the invention and cilomilast,
a compound of the invention and tetomilast, or
a compound of the invention and oglemilast.

In a further preferred embodiment, the pharmaceutical composition comprises a compound of the invention in combination with an antidepressant. In a particularly preferred embodiment, the pharmaceutical composition comprises:
a compound of the invention and bupropion.

In a further preferred embodiment, the pharmaceutical composition comprises a compound of the invention in combination with an antibiotic. In a particularly preferred embodiment, the pharmaceutical composition comprises:
a compound of the invention and amoxicillin,
a compound of the invention and ampicillin,
a compound of the invention and levofloxacin,
a compound of the invention and clarithromycin,
a compound of the invention and ciprofloxacin,
a compound of the invention and amoxicillin,
a compound of the invention and telithromycin, or
a compound of the invention and azithromycin.

In a further preferred embodiment, the pharmaceutical composition comprises a compound of the invention in combination with a corticosteroid and a beta-mimetic. In a particularly preferred embodiment, the pharmaceutical composition comprises:
a compound of the invention, budesonide and indacaterol,
a compound of the invention, budesonide and formoterol,
a compound of the invention, budesonide and salmeterol,
a compound of the invention, fluticasone and indacaterol,
a compound of the invention, fluticasone and formoterol,
a compound of the invention, fluticasone and salmeterol,
a compound of the invention, beclometasone and indacaterol,
a compound of the invention, beclometasone and formoterol,
a compound of the invention, beclometasone and salmeterol,
a compound of the invention, triamcinolone acetonide and indacaterol,
a compound of the invention, triamcinolone acetonide and formoterol,
a compound of the invention, triamcinolone acetonide and salmeterol,
a compound of the invention, ciclesonide and indacaterol,
a compound of the invention, ciclesonide and formoterol, or
a compound of the invention, ciclesonide and salmeterol.

In a further preferred embodiment, the pharmaceutical composition comprises a compound of the invention in combination with a corticosteroid and an anticholinergic. In a particularly preferred embodiment, the pharmaceutical composition comprises:
a compound of the invention, budesonide and tiotropium bromide,
a compound of the invention, budesonide and ipratropium bromide,
a compound of the invention, fluticasone and tiotropium bromide,
a compound of the invention, fluticasone and ipratropium bromide,
a compound of the invention, beclometasone and tiotropium bromide,
a compound of the invention, beclometasone and ipratropium bromide,
a compound of the invention, triamcinolone acetonide and tiotropium bromide,
a compound of the invention, triamcinolone acetonide and ipratropium bromide,
a compound of the invention, ciclesonide and tiotropium bromide, or
a compound of the invention, ciclesonide and ipratropium bromide.

The above mentioned compound of the invention is preferably a compound according to the

### examples.

The invention furthermore relates to pharmaceutical compositions according to the invention, as defined above, inhibiting the type 5 phosphodiesterase, especially for the treatment or prophylaxis of diseases alleviated by inhibition of type 5 phosphodiesterase, in particular for the treatment or prophylaxis of the diseases exemplified above.

The invention also encompasses pharmaceutical compositions according to the invention, as defined above, for the treatment or prophylaxis of the following diseases: acute and chronic airway diseases, such as pulmonary hypertension, lung fibrosis, asthma, bronchitis, emphysema and chronic obstructive pulmonary disease; portal hypertension, liver cirrhosis, toxic liver damage, hepatitis, non-alcoholic steatohepatitis and liver fibrosis.

The pharmaceutical compositions according to the invention preferably contain the compound or compounds of the invention in a total amount of from 0.1 to 99.9 wt%, more preferably 5 to 95 wt%, in particular 20 to 80 wt%. In case at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics is present in the pharmaceutical compositions of the invention, the total amount of said therapeutic agent or therapeutic agents in the pharmaceutical compositions is preferably in the range of from 0.1 to 99.9 wt%, more preferably 5 to 95 wt%, in particular 20 to 80 wt%, under the provision that the total amount of the compound or compounds of the invention and the therapeutic agent or therapeutic agents is less than 100 wt%. Preferably, the at least one compound of the invention and the at least one therapeutic agent are present in the pharmaceutical composition in a weight ratio of from 1000 : 1 to 1: 1000.

As pharmaceutically acceptable auxiliaries, any auxiliaries known to be suitable for preparing pharmaceutical compositions can be used. Examples thereof include, but are not limited to, solvents, excipients, dispersants, emulsifiers, solubilizers, gel formers, ointment bases, antioxidants, preservatives, stabilizers, carriers, fillers, binders, thickeners, complexing agents, disintegrating agents, buffers, permeation promoters, polymers, lubricants, coating agents, propellants, tonicity adjusting agents, surfactants, colorants, flavorings, sweeteners and dyes. In particular, auxiliaries of a type appropriate to the desired formulation and the desired mode of administration are used.

The pharmaceutical compositions can be formulated, for example, into tablets, coated tablets (dragees), pills, cachets, capsules (caplets), granules, powders, suppositories, solutions (e.g., but not limited to, sterile solutions), emulsions, suspensions, ointments, creams, lotions, pastes, oils, gels, sprays and patches (e.g., but not limited to, transdermal therapeutic systems). Additionally, the pharmaceutical compositions can be prepared as e.g. liposome delivery systems, systems in which the compound of the invention is coupled to monoclonal antibodies and systems in which the compound of the invention is coupled to polymers (e.g., but not limited to, soluble or biodegradable polymers).

In case of pharmaceutical compositions comprising at least one of the compounds of the invention and at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics, the compound of the invention and the therapeutic agent may be formulated together into the same dosage form (e.g., but not limited to, tablets), separately into the same dosage form (e.g., but not limited to, tablets), or into different dosage forms (without limitation e.g. the compound of the invention may be formulated as tablet and the therapeutic agent may be formulated as powder, solution or suspension).

The pharmaceutical compositions can be manufactured in a manner known to a person skilled in the art, e.g. by dissolving, mixing, granulating, dragee-making, levigating, emulsifying, encapsulating, entrapping or lyophilizing processes.

The selected formulation depends inter alia on the route of administering the pharmaceutical composition. The pharmaceutical compositions of the invention can be administered by any suitable route, for example, by the oral, sublingual, buccal, intravenous, intraarterial, intramuscular, subcutaneous, intracutaneous, topical, transdermal, intranasal, intraocular, intraperitoneal, intrasternal, intracoronary, transurethral, rectal or vaginal route, by inhalation or by insufflation. Oral administration is preferred.

In case of pharmaceutical compositions comprising at least one of the compounds of the invention and at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics, the compound of the invention and the therapeutic agent may be administered by the same route, e.g., without limitation, orally, or by different routes, e.g., without limitation, the compound of the invention can be administered orally and the therapeutic agent can be administered by inhalation or instillation.

Tablets, coated tablets (dragees), pills, cachets, capsules (caplets), granules, solutions, emulsions and suspensions are e.g. suitable for oral administration. In particular, said formulations can be adapted so as to represent, for example, an enteric form, an immediate release form, a delayed release form, a repeated dose release form, a prolonged release form or a sustained release form. Said forms can be obtained, for example, by coating tablets, by dividing tablets into several compartments separated by layers disintegrating under different conditions (e.g. pH conditions) or by coupling the compound of the invention to a biodegradable polymer.

Administration by inhalation or instillation is preferably made by using an aerosol. The aerosol is a liquid-gaseous dispersion, a solid-gaseous dispersion or a mixed liquid/solid-gaseous dispersion.

The aerosol may be generated by means of aerosol-producing devices such as dry powder inhalers (DPIs), pressurized metered dose inhalers (PMDIs) and nebulizers. Depending on the kind of the compound of the invention, and optionally the therapeutic agent, to be administered, the aerosol-producing device can contain the compound and, optionally, the therapeutic agent in form of a powder, a solution or a dispersion. The powder may contain, for example, one or more of the following auxiliaries: carriers, stabilizers and fillers. The solution may contain in addition to the solvent, for example, one or more of the following auxiliaries: propellants, solubilizers (co-solvents), surfactants, stabilizers, buffers, tonicity adjusting agents, preservatives and flavorings. The dispersion may contain in addition to the dispersant, for example, one or more of the following auxiliaries: propellants, surfactants, stabilizers, buffers, preservatives and flavorings. Examples of carriers include, but are not limited to, saccharides, e.g. lactose and glucose. Examples of propellants include, but are not limited to, fluorohydrocarbons, e.g. 1,1,1,2-tetrafluoroethane and 1,1,1,2,3,3,3-heptafluoropropane.

The particle size of the aerosol particles (solid, liquid or solid/liquid particles) is preferably less than 100 µm, more preferably it is in the range of from 0.5 to 10 µm, in particular in the range of from 2 to 6 µm (D50 value, measured by laser diffraction).

Specific aerosol-producing devices which may be used for inhaled administration include, but are not limited to, Cyclohaler®, Diskhaler®, Rotadisk®, Turbohaler®, Autohaler®, Turbohaler®, Novolizer®, Easyhaler®, Aerolizer®, Jethaler®, Diskus®, Ultrahaler® and Mystic® inhalers. The aerosol-producing devices may be combined with spacers or expanders, e.g. Aerochamber®, Nebulator®, Volumatic® and Rondo®, for improving inhalation efficiency.

In case of topical administration, suitable pharmaceutical formulations are, for example, ointments, creams, lotions, pastes, gels, powders, solutions, emulsions, suspensions, oils, sprays and patches (e.g., but not limited to, transdermal therapeutic systems).

For parenteral modes of administration such as, for example, intravenous, intraarterial, intramuscular, subcutaneous, intracutaneous, intraperitoneal and intrasternal administration, preferably solutions (e.g., but not limited to, sterile solutions, isotonic solutions) are used. They are preferably administered by injection or infusion techniques.

In case of intranasal administration, for example, sprays and solutions to be applied in drop form are preferred formulations.

For intraocular administration, solutions to be applied in drop form, gels and ointments are exemplified formulations.

Generally, the pharmaceutical compositions according to the invention can be administered such that the dose of the compound of the invention is in the range customary for type 5 phosphodiesterase inhibitors. In particular, a dose in the range of from 0.01 to 4000 mg of the compound of the invention per day is preferred for an average adult patient having a body weight of 70 kg. In this respect, it is to be noted that the dose is dependent, for example, on the specific compound used, the species treated, age, body weight, general health, sex and diet of the subject treated, mode and time of administration, rate of excretion, severity of the disease to be treated and drug combination. In case the pharmaceutical composition of the invention comprises at least one of the compounds of the invention and at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics, the same dose ranges apply to the therapeutic agent.

The pharmaceutical compositions according to the invention can be administered in a single dose per day or in multiple subdoses, for example, 2 to 4 doses per day. A single dose unit of the pharmaceutical composition can contain e.g. from 0.01 mg to 4000 mg, preferably 0.1 mg to 2000 mg, more preferably 0.5 to 1000 mg, most preferably 1 to 500 mg, of the compound of the invention. In case the pharmaceutical composition of the invention comprises at least one of the compounds of the invention and at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics, a single dose unit of the pharmaceutical composition can contain e.g. from 0.01 mg to 4000 mg, preferably 0.1 mg to 2000 mg, more preferably 0.5 to 1000 mg, most preferably 1 to 500 mg, of the therapeutic agent. Furthermore, the pharmaceutical composition can be adapted to weekly, monthly or even more infrequent administration, for example by using an implant, e.g. a subcutaneous or intramuscular implant, by using the compound of the invention in form of a sparingly soluble salt or by using the compound of the invention coupled to a polymer. Administration of the pharmaceutical composition in a single dose per day is preferred.

In case the pharmaceutical composition of the invention comprises at least one of the compounds of the invention and at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics, administration of the compound of the invention and administration of the therapeutic agent can be made simultaneously or sequentially. In case of sequential administration, the compound of the invention can be administered before or after administration of the therapeutic agent.

Furthermore, the invention relates to the compound 6-benzyl-3,3-dimethyl-2,3,4,7-tetrahydro-indolo[2,3-c]quinolin-1-one for use in the treatment or prophylaxis of diseases, especially diseases alleviated by inhibition of the type 5 phosphodiesterase. In particular, the invention relates to the compound 6-benzyl-3,3-dimethyl-2,3,4,7-tetrahydro-indolo[2,3-c]quinolin-1-one for use in the treatment or prophylaxis of the diseases exemplified above.

The invention further relates to the compound 6-benzyl-3,3-dimethyl-2,3,4,7-tetrahydro-indolo[2,3-c]quinolin-1-one for use in the treatment or prophylaxis of the following diseases: acute and chronic airway diseases, such as pulmonary hypertension, lung fibrosis, asthma, bronchitis, emphysema and chronic obstructive pulmonary disease; portal hypertension, liver cirrhosis, toxic liver damage, hepatitis, non-alcoholic steatohepatitis and liver fibrosis.

The invention also relates to the use of the compound 6-benzyl-3,3-dimethyl-2,3,4,7-tetrahydro-indolo[2,3-c]quinolin-1-one in the manufacture of a pharmaceutical composition inhibiting the type 5 phosphodiesterase, in particular a pharmaceutical composition for the treatment or prophylaxis of diseases alleviated by inhibition of the type 5 phosphodiesterase, preferably, a pharmaceutical composition for the treatment or prophylaxis of the diseases exemplified above.

In particular, the invention relates to the use of the compound 6-benzyl-3,3-dimethyl-2,3,4,7-tetra-hydro-indolo[2,3-c]quinolin-1-one in the manufacture of a pharmaceutical composition for the treatment or prophylaxis of an acute or chronic airway disease, such as, but not limited to, pulmonary hypertension, lung fibrosis, asthma, bronchitis, emphysema and chronic obstructive pulmonary disease.

Furthermore, the invention relates to the use of the compound 6-benzyl-3,3-dimethyl-2,3,4,7-tetra-hydro-indolo[2,3-c]quinolin-1-one in the manufacture of a pharmaceutical composition for the treatment or prophylaxis of portal hypertension, liver cirrhosis, toxic liver damage, hepatitis, non-alcoholic steatohepatitis or liver fibrosis.

The invention further relates to a method of treating or preventing a disease comprising administering to a patient in need thereof a therapeutically effective amount of the compound 6-benzyl-3,3-dimethyl-2,3,4,7-tetrahydro-indolo[2,3-c]quinolin-1-one.

In particular, the invention relates to a method of treating or preventing one of the above mentioned diseases comprising administering to a patient in need thereof a therapeutically effective amount of the compound 6-benzyl-3,3-dimethyl-2,3,4,7-tetrahydro-indolo[2,3-c]quinolin-1-one.

Especially, the invention relates to a method of treating or preventing a disease which is alleviated by inhibition of the type 5 phosphodiesterase comprising administering to a patient in need thereof a therapeutically effective amount of the compound 6-benzyl-3,3-dimethyl-2,3,4,7-tetrahydro-indolo[2,3-c]quinolin-1-one.

Preferably, the invention relates to a method of treating or preventing an acute or chronic airway disease, for example, but not limited to, pulmonary hypertension, lung fibrosis, asthma, bronchitis, emphysema and chronic obstructive pulmonary disease, comprising administering to a patient in need thereof a therapeutically effective amount of the compound 6-benzyl-3,3-dimethyl-2,3,4,7-tetrahydro-indolo[2,3-c]quinolin-1-one.

Furthermore, the invention preferably relates to a method of treating or preventing portal hypertension, liver cirrhosis, toxic liver damage, hepatitis, non-alcoholic steatohepatitis or liver fibrosis comprising administering to a patient in need thereof a therapeutically effective amount of the compound 6-benzyl-3,3-dimethyl-2,3,4,7-tetrahydro-indolo[2,3-c]quinolin-1-one.

The invention furthermore relates to a pharmaceutical composition which comprises the compound 6-benzyl-3,3-dimethyl-2,3,4,7-tetrahydro-indolo[2,3-c]quinolin-1-one together with at least one pharmaceutically acceptable auxiliary.

The invention additionally relates to a pharmaceutical composition comprising the compound 6-benzyl-3,3-dimethyl-2,3,4,7-tetrahydro-indolo[2,3-c]quinolin-1-one, at least one pharmaceutically acceptable auxiliary and at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics.

### Biological investigations

### Method for measuring inhibition of PDE5 activity:

As a source for human PDE5, platelets were used. For that purpose, 150 ml fresh blood from human donors anticoagulated with citrate [final concentration 0.3% (w/v)] was centrifuged at 200 g for 10 min to obtain the so-called platelet-rich-plasma (PRP) as a supernatant. 1/10 volume of ACD solution (85 mM Na₃-citrate, 111 mM D-glucose, 71 mM citric acid, pH 4.4) was added to 9/10 volume of PRP. After centrifugation (1,400 g, 10 min) the cell pellet was resuspended in 3 ml homogenization buffer (NaCl 140 mM, KCl 3.8 mM, EGTA 1mM, MgCl₂ 1mM, Tris-HCl 20 mM, beta-mercaptoethanol 1mM, pH 8.2) plus protease-inhibitor mix giving rise to the final concentrations of 0.5 mM Pefablock (Roche), 10 µM Leupeptin, 5 µM Trypsininhibitor, 2 mM Benzamidin and 10 µM Pepstatin A. The suspension was sonified and thereafter centrifuged for 15 min at 10,000 g. The resulting supernatant (platelet lysate) was used for enzymatic testings.

PDE5A1 activity is inhibited by the compounds of the invention in a modified SPA (scintillation proximity assay) test, supplied by Amersham Biosciences (see procedural instructions "phosphodiesterase [3H]cAMP SPA enzyme assay, code TRKQ 7090"), carried out in 96-well microtitre plates (MTP's). The test volume is 100 µl and contains 20 mM Tris buffer (pH 7.4), 0.1 mg of BSA (bovine serum albumin)/ml, 5 mM Mg²⁺, 1 µM motapizone, 10 nM PDE2 inhibitor BAY-60-7550, 0.5 µM cGMP (including about 50,000 cpm of [3H]cGMP as a tracer), 1 µl of the respective compound dilution in dimethylsulfoxide (DMSO) and sufficient PDE5-containing platelet lysat (10,000xg supernatant, see above) to ensure that 10-20 wt% of the cGMP is converted under the said experimental conditions. The final concentration of DMSO in the assay (1% v/v) does not substantially affect the activity of the PDE investigated. After a preincubation of 5 min at 37°C, the reaction was started by adding the substrate (cGMP) and the assay was incubated for a further 15 min; after that, it was stopped by adding SPA beads (50 µl). In accordance with the manufacturer's instructions, the SPA beads had previously been resuspended in water, but were then diluted 1:3 (v/v) in water; the diluted solution also contains 3 mM 8-methoxymethyl-3-isobutyl-1-methylxanthine (IBMX) to ensure a complete PDE activity stop. After the beads have been sedimented (> 30 min), the MTP's are analyzed in commercially available luminescence detection devices. The corresponding IC₅₀ values of the compounds for the inhibition of PDE activity are determined from the concentration-effect curves by means of non-linear regression.

Representative inhibitory values determined for the compounds of the invention are given in the following Table:

| Example | -log IC₅₀ (mol/l) |
|---|---|
| 1 | 8.52 |
| 2 | 8.20 |
| 3 | 7.71 |
| 4 | 8.47 |
| 5 | 7.97 |
| 6 | 7.90 |
| 7 | 8.41 |
| 8 | 7.09 |
| 9 | 7.64 |
| 10 | 7.10 |
| 11 | 6.87 |
| 12 | 7.57 |
| 13 | 6.87 |
| 14 | 6.46 |
| 15 | 8.20 |
| 17 | 8.50 |
| 18 | 8.44 |
| 19 | 6.54 |
| 20 | 7.99 |
| 21 | 6.90 |
| 22 | 7.81 |
| 23 | 8.90 |
| 24 | 7.85 |
| 25 | 8.53 |
| 26 | 6.45 |
| 27 | 8.65 |
| 28 | 7.74 |
| 29 | 8.90 |
| 30 | 8.40 |
| 31 | 9.40 |
| 32 | 9.72 |
| 34 | 9.20 |
| 35 | 8.72 |
| 36 | 7.93 |
| 37 | 8.29 |
| 38 | 7.59 |
| 39 | 7.94 |
| 40 | 7.78 |
| 41 | 7.46 |
| 42 | 7.17 |
| 43 | 8.27 |
| 44 | 7.88 |
| 45 | 7.18 |
| 47 | 8.26 |
| 48 | 7.69 |
| 49 | 7.05 |
| 50 | 7.57 |
| 51 | 7.60 |
| 52 | 6.72 |
| 53 | 8.39 |
| 54 | 6.83 |
| 55 | 8.57 |
| 56 | 7.15 |
| 57 | 8.14 |
| 58 | 7.30 |
| 59 | 7.33 |
| 60 | 7.54 |
| 61 | 8.95 |
| 62 | 7.78 |
| 63 | 8.10 |
| 64 | 8.80 |
| 65 | 6.52 |
| 66 | 7.64 |
| 67 | 8.60 |
| 68 | 7.31 |
| 69 | 9.70 |
| 70 | 9.49 |
| 71 | 7.98 |
| 72 | 7.38 |
| 73 | 8.20 |
| 74 | 7.81 |
| 75 | 9.10 |
| 76 | 7.00 |
| 77 | 9.00 |
| 78 | 7.50 |
| 79 | 7.60 |
| 80 | 6.40 |
| 81 | 8.70 |
| 82 | 8.50 |

## Claims

1. Compound of formula (I) 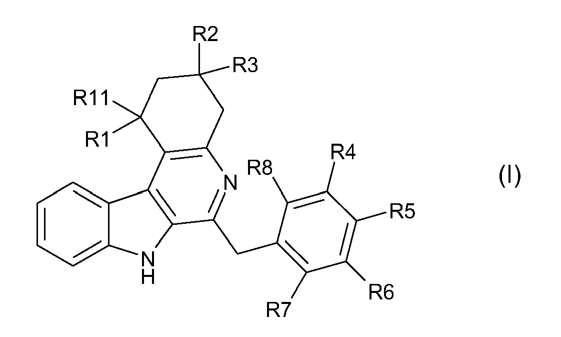 wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl, -NH-C(O)-NH₂ and a methoxy group substituted by 2 or 3 fluorine atoms; or
R4 and R5 combine to form a group selected from -O-CH₂-O-, -O-CH₂-CH₂- and -CH₂-CH₂-O-;
R6 is selected from the group consisting of hydrogen and halogen;
R7 is selected from the group consisting of hydrogen and halogen;
R8 is selected from the group consisting of hydrogen and halogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof or a stereoisomer of the compound, the salt, the N-oxide of the compound or the N-oxide of the salt thereof.

2. Compound according to claim 1, wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen, 1-3C-alkoxy, nitro and amino;
R5 is selected from the group consisting of hydrogen, halogen, 1-3C-alkyl, hydroxy, 1-3C-alkoxy, nitro, amino, -NH-C(O)-1-2C-alkyl and -NH-C(O)-NH₂;
R6 is selected from the group consisting of hydrogen and halogen;
R7 is selected from the group consisting of hydrogen and halogen;
R8 is selected from the group consisting of hydrogen and halogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof or a stereoisomer of the compound, the salt, the N-oxide of the compound or the N-oxide of the salt thereof.

3. Compound according to claim 1 or 2, wherein
R1 is selected from the group consisting of hydrogen and hydroxy;
R11 is hydrogen; or
R1 and R11 combine to form an oxo group;
R2 is selected from the group consisting of hydrogen and 1-3C-alkyl;
R3 is selected from the group consisting of hydrogen and 1-3C-alkyl;
R4 is selected from the group consisting of hydrogen, halogen and 1-3C-alkoxy;
R5 is selected from the group consisting of hydrogen, halogen, 1-3C-alkyl, hydroxy and 1-3C-alkoxy;
R6 is selected from the group consisting of hydrogen and halogen;
R7 is selected from the group consisting of hydrogen and halogen;
R8 is selected from the group consisting of hydrogen and halogen;
under the proviso that the compound 6-benzyl-3,3-dimethyl-2,3,4,7-tetrahydro-indolo[2,3-c]quinolin-1-one is disclaimed;
a salt thereof, an N-oxide of the compound or the salt thereof or a stereoisomer of the compound, the salt, the N-oxide of the compound or the N-oxide of the salt thereof.

4. Compound according to claim 1 selected from the group consisting of
6-(4-Methoxy-benzyl)-3,3-dimethyl-2,3,4,7-tetrahydro-indolo[2,3-c]quinolin-1-one;
6-(4-Methoxy-benzyl)-2,3,4,7-tetrahydro-indolo[2,3-c]quinolin-1-one;
6-Benzyl-2,3,4,7-tetrahydro-indolo[2,3-c]quinolin-1-one;
6-(4-Methoxy-benzyl)-3-methyl-2,3,4,7-tetrahydro-1H-indolo[2,3-c]quinoline;
6-(4-Bromo-benzyl)-3,3-dimethyl-2,3,4,7-tetrahydro-indolo[2,3-c]quinolin-1-one;
6-(4-Methoxy-benzyl)-2,3,4,7-tetrahydro-1H-indolo[2,3-c]quinoline;
6-Benzo[1,3]dioxol-5-ylmethyl-3,3-dimethyl-2,3,4,7-tetrahydro-indolo[2,3-c]quinolin-1-one;
6-(4-Bromo-benzyl)-2,3,4,7-tetrahydro-1H-indolo[2,3-c]quinoline;
6-(3-Methoxy-benzyl)-3,3-dimethyl-2,3,4,7-tetrahydro-indolo[2,3-c]quinolin-1-one;
3,3-Dimethyl-6-(4-trifluoromethoxy-benzyl)-2,3,4,7-tetrahydro-indolo[2,3-c]quinolin-1-one;
6-(4-Ethoxy-benzyl)-3,3-dimethyl-2,3,4,7-tetrahydro-indolo[2,3-c]quinolin-1-one;
3,3-Dimethyl-6-(3-nitro-benzyl)-2,3,4,7-tetrahydro-indolo[2,3-c]quinolin-1-one;
6-(4-Isopropyl-benzyl)-3,3-dimethyl-2,3,4,7-tetrahydro-indolo[2,3-c]quinolin-1-one;
6-(3-Chloro-4-methoxy-benzyl)-3,3-dimethyl-2,3,4,7-tetrahydro-indolo[2,3-c]quinolin-1-one;
3,3-Dimethyl-6-(4-nitro-benzyl)-2,3,4,7-tetrahydro-indolo[2,3-c]quinolin-1-one;
6-(4-Methoxy-benzyl)-3-methyl-2,3,4,7-tetrahydro-indolo[2,3-c]quinolin-1-one;
6-Benzo[1,3]dioxol-5-ylmethyl-2,3,4,7-tetrahydro-indolo[2,3-c]quinolin-1-one;
6-(3-Nitro-benzyl)-2,3,4,7-tetrahydro-indolo[2,3-c]quinolin-1-one;
6-(4-Chloro-benzyl)-3,3-dimethyl-2,3,4,7-tetrahydro-indolo[2,3-c]quinolin-1-one;
6-(3-Bromo-benzyl)-3,3-dimethyl-2,3,4,7-tetrahydro-indolo[2,3-c]quinolin-1-one;
6-(3,5-Difluoro-benzyl)-3,3-dimethyl-2,3,4,7-tetrahydro-indolo[2,3-c]quinolin-1-one;
6-(3-Fluoro-4-methoxy-benzyl)-3,3-dimethyl-2,3,4,7-tetrahydro-indolo[2,3-c]quinolin-1-one;
6-(3,4-Difluoro-benzyl)-3,3-dimethyl-2,3,4,7-tetrahydro-indolo[2,3-c]quinolin-1-one;
3,3-Dimethyl-6-(4-methyl-benzyl)-2,3,4,7-tetrahydro-indolo[2,3-c]quinolin-1-one;
6-(4-Nitro-benzyl)-2,3,4,7-tetrahydro-indolo[2,3-c]quinolin-1-one;
6-(2-Fluoro-4-methoxy-benzyl)-3,3-dimethyl-2,3,4,7-tetrahydro-indolo[2,3-c]quinolin-1-one;
6-(4-Fluoro-benzyl)-3,3-dimethyl-2,3,4,7-tetrahydro-indolo[2,3-c]quinolin-1-one;
6-(3-Fluoro-4-methoxy-benzyl)-2,3,4,7-tetrahydro-indolo[2,3-c]quinolin-1-one;
6-(3-Fluoro-4-methoxy-benzyl)-2,3,4,7-tetrahydro-1H-indolo[2,3-c]quinoline;
6-(2,3-Difluoro-4-methoxy-benzyl)-3,3-dimethyl-2,3,4,7-tetrahydro-indolo[2,3-c]quinolin-1-one;
6-(3,5-Difluoro-4-methoxy-benzyl)-3,3-dimethyl-2,3,4,7-tetrahydro-indolo[2,3-c]quinolin-1-one;
6-(2,3-Dihydro-benzofuran-5-ylmethyl)-3,3-dimethyl-2,3,4,7-tetrahydro-indolo[2,3-c]quinolin-1-one;
6-(3-Fluoro-4-hydroxy-benzyl)-3,3-dimethyl-2,3,4,7-tetrahydro-indolo[2,3-c]quinolin-1-one;
6-(4-Hydroxy-benzyl)-3,3-dimethyl-2,3,4,7-tetrahydro-indolo[2,3-c]quinolin-1-one;
6-(4-Amino-benzyl)-3,3-dimethyl-2,3,4,7-tetrahydro-indolo[2,3-c]quinolin-1-one;
6-(3-Amino-benzyl)-3,3-dimethyl-2,3,4,7-tetrahydro-indolo[2,3-c]quinolin-1-one;
6-(4-Amino-benzyl)-2,3,4,7-tetrahydro-indolo[2,3-c]quinolin-1-one;
6-(3-Amino-benzyl)-2,3,4,7-tetrahydro-indolo[2,3-c]quinolin-1-one;
N-[4-(3,3-Dimethyl-1-oxo-2,3,4,7-tetrahydro-1H-indolo[2,3-c]quinolin-6-ylmethyl)-phenyl]-acetamide;
N-[4-(3,3-Dimethyl-1-oxo-2,3,4,7-tetrahydro-1H-indolo[2,3-c]quinolin-6-ylmethyl)-phenyl]-propionamide;
N-[4-(1-Oxo-2,3,4,7-tetrahydro-1H-indolo[2,3-c]quinolin-6-ylmethyl)-phenyl]-acetamide;
6-(4-Methoxy-benzyl)-3,3-dimethyl-2,3,4,7-tetrahydro-1H-indolo[2,3-c]quinolin-1-ol;
6-(4-Methoxy-benzyl)-2,3,4,7-tetrahydro-1H-indolo[2,3-c]quinolin-1-ol;
6-Benzyl-2,3,4,7-tetrahydro-1H-indolo[2,3-c]quinolin-1-ol;
6-Benzyl-3,3-dimethyl-2,3,4,7-tetrahydro-1H-indolo[2,3-c]quinolin-1-ol;
6-(4-Hydroxy-benzyl)-3,3-dimethyl-2,3,4,7-tetrahydro-1H-indolo[2,3-c]quinolin-1-ol;
3,3-Dimethyl-6-(4-trifluoromethoxy-benzyl)-2,3,4,7-tetrahydro-1H-indolo[2,3-c]quinolin-1-ol;
6-(4-Amino-benzyl)-3,3-dimethyl-2,3,4,7-tetrahydro-1H-indolo[2,3-c]quinolin-1-ol;
6-(4-Ethoxy-benzyl)-3,3-dimethyl-2,3,4,7-tetrahydro-1H-indolo[2,3-c]quinolin-1-ol;
3,3-Dimethyl-6-(3-nitro-benzyl)-2,3,4,7-tetrahydro-1H-indolo[2,3-c]quinolin-1-ol;
6-(3-Amino-benzyl)-3,3-dimethyl-2,3,4,7-tetrahydro-1H-indolo[2,3-c]quinolin-1-ol;
6-(4-Isopropyl-benzyl)-3,3-dimethyl-2,3,4,7-tetrahydro-1H-indolo[2,3-c]quinolin-1-ol;
6-Benzo[1,3]dioxol-5-ylmethyl-3,3-dimethyl-2,3,4,7-tetrahydro-1H-indolo[2,3-c]quinolin-1-ol;
N-[4-(1-Hydroxy-3,3-dimethyl-2,3,4,7-tetrahydro-1H-indolo[2,3-c]quinolin-6-ylmethyl)-phenyl]-propionamide;
6-Benzo[1,3]dioxol-5-ylmethyl-2,3,4,7-tetrahydro-1H-indolo[2,3-c]quinolin-1-ol;
6-(3-Bromo-benzyl)-3,3-dimethyl-2,3,4,7-tetrahydro-1H-indolo[2,3-c]quinolin-1-ol;
N-[4-(1-Hydroxy-3,3-dimethyl-2,3,4,7-tetrahydro-1H-indolo[2,3-c]quinolin-6-ylmethyl)-phenyl]-acetamide;
6-(4-Fluoro-benzyl)-3,3-dimethyl-2,3,4,7-tetrahydro-1H-indolo[2,3-c]quinolin-1-ol;
6-(2-Fluoro-4-methoxy-benzyl)-3,3-dimethyl-2,3,4,7-tetrahydro-1H-indolo[2,3-c]quinolin-1-ol;
6-(3,4-Difluoro-benzyl)-3,3-dimethyl-2,3,4,7-tetrahydro-1H-indolo[2,3-c]quinolin-1-ol;
6-(3,5-Difluoro-benzyl)-3,3-dimethyl-2,3,4,7-tetrahydro-1H-indolo[2,3-c]quinolin-1-ol;
6-(3-Fluoro-4-methoxy-benzyl)-3,3-dimethyl-2,3,4,7-tetrahydro-1H-indolo[2,3-c]quinolin-1-ol;
N-[4-(1-Hydroxy-2,3,4,7-tetrahydro-1H-indolo[2,3-c]quinolin-6-ylmethyl)-phenyl]-acetamide;
[4-(1-Hydroxy-3,3-dimethyl-2,3,4,7-tetrahydro-1H-indolo[2,3-c]quinolin-6-ylmethyl)-phenyl]-urea;
6-(3-Fluoro-4-methoxy-benzyl)-2,3,4,7-tetrahydro-1H-indolo[2,3-c]quinolin-1-ol;
6-(3-Amino-benzyl)-2,3,4,7-tetrahydro-1H-indolo[2,3-c]quinolin-1-ol;
6-(3,5-Difluoro-4-methoxy-benzyl)-3,3-dimethyl-2,3,4,7-tetrahydro-1H-indolo[2,3-c]quinolin-1-ol;
6-(2,3-Difluoro-4-methoxy-benzyl)-3,3-dimethyl-2,3,4,7-tetrahydro-1H-indolo[2,3-c]quinolin-1-ol;
[4-(3,3-Dimethyl-1-oxo-2,3,4,7-tetrahydro-1 H-indolo[2,3-c]quinolin-6-ylmethyl)-phenyl]-urea;
[4-(1-Oxo-2,3,4,7-tetrahydro-1H-indolo[2,3-c]quinolin-6-ylmethyl)-phenyl]-urea;
6-(4-Methoxy-benzyl)-3,3-dimethyl-2,3,4,7-tetrahydro-1H-indolo[2,3-c]quinoline;
6-(3-Fluoro-4-hydroxy-benzyl)-3,3-dimethyl-2,3,4,7-tetrahydro-1H-indolo[2,3-c]quinolin-1-ol;
6-(2,3-Dihydro-benzofuran-5-ylmethyl)-3,3-dimethyl-2,3,4,7-tetrahydro-1H-indolo[2,3-c]quinolin-1-ol;
a salt thereof, an N-oxide of the compound or the salt thereof and a stereoisomer of the compound, the salt, the N-oxide of the compound or the N-oxide of the salt thereof.

5. Compound, pharmaceutically acceptable salt thereof, N-oxide of the compound or the salt thereof or stereoisomer of the compound, the salt, the N-oxide of the compound or the N-oxide of the salt thereof according to any of claims 1 to 4 for use in the treatment or prophylaxis of diseases.

6. Pharmaceutical composition comprising at least one of the compounds, pharmaceutically acceptable salts thereof, N-oxides of the compounds and the salts thereof and stereoisomers of the compounds, salts, N-oxides of the compounds and N-oxides of the salts thereof according to any of claims 1 to 4 together with at least one pharmaceutically acceptable auxiliary.

7. Pharmaceutical composition according to claim 6 further comprising at least one therapeutic agent selected from the group consisting of corticosteroids, anticholinergics, beta-mimetics, lung surfactants, endothelin antagonists, prostacyclins, calcium channel blockers, beta-blockers, type 4 phosphodiesterase inhibitors, antidepressants and antibiotics.

8. Use of a compound, pharmaceutically acceptable salt thereof, N-oxide of the compound or the salt thereof or stereoisomer of the compound, the salt, the N-oxide of the compound or the N-oxide of the salt thereof according to any of claims 1 to 4 in the manufacture of a pharmaceutical composition inhibiting the type 5 phosphodiesterase.

9. Use of a compound, pharmaceutically acceptable salt thereof, N-oxide of the compound or the salt thereof or stereoisomer of the compound, the salt, the N-oxide of the compound or the N-oxide of the salt thereof according to any of claims 1 to 4 in the manufacture of a pharmaceutical composition for the treatment or prophylaxis of an acute or chronic airway disease.

10. Use according to claim 9, wherein the acute or chronic airway disease is selected from the group consisting of pulmonary hypertension, lung fibrosis, asthma, bronchitis, emphysema and chronic obstructive pulmonary disease.

11. Use of a compound, pharmaceutically acceptable salt thereof, N-oxide of the compound or the salt thereof or stereoisomer of the compound, the salt, the N-oxide of the compound or the N-oxide of the salt thereof according to any of claims 1 to 4 in the manufacture of a pharmaceutical composition for the treatment or prophylaxis of portal hypertension, liver cirrhosis, toxic liver damage, hepatitis, non-alcoholic steatohepatitis or liver fibrosis.

12. Method of treating or preventing an acute or chronic airway disease comprising administering to a patient in need thereof a therapeutically effective amount of a compound, pharmaceutically acceptable salt thereof, N-oxide of the compound or the salt thereof or stereoisomer of the compound, the salt, the N-oxide of the compound or the N-oxide of the salt thereof according to any of claims 1 to 4.

13. Method of treating or preventing an acute or chronic airway disease according to claim 12, in which the acute or chronic airway disease is selected from the group consisting of pulmonary hypertension, lung fibrosis, asthma, bronchitis, emphysema and chronic obstructive pulmonary disease.

14. Method of treating or preventing portal hypertension, liver cirrhosis, toxic liver damage, hepatitis, non-alcoholic steatohepatitis or liver fibrosis comprising administering to a patient in need thereof a therapeutically effective amount of a compound, pharmaceutically acceptable salt thereof, N-oxide of the compound or the salt thereof or stereoisomer of the compound, the salt, the N-oxide of the compound or the N-oxide of the salt thereof according to any of claims 1 to 4.
